# EUROPEAN PATENT SPECIFICATION

(11) **EP 2 723 770 B1**
(45) Date of publication and mention of the grant of the patent: **31.07.2019**
(21) Application number: 12735242.5
(22) Date of filing: 25.06.2012
(51) Int. Cl.: C07K 16/00, C07K 16/28

(54) **FC VARIANTS WITH REDUCED EFFECTOR FUNCTIONS**
FC-VARIANTEN MIT REDUZIERTEN EFFEKTORFUNKTIONEN
VARIANTS FC À FONCTIONS EFFECTRICES RÉDUITES

(30) Priority: 24.06.2011 EP 11305811
(43) Date of publication of application: 30.04.2014
(73) Proprietor: Laboratoire Français du Fractionnement et des Biotechnologies, 91940 Les Ulis (FR)
(72) Inventor: FONTAYNE, Alexandre, F-59110 La Madeleine (FR); JORIEUX, Sylvie, 59160 LOMME (FR); MONNET-MARS, Céline, F-31700 Blagnac (FR); MONDON, Philippe, F-31450 Donneville (FR); KHARRAT, Abdelhakim, F-31450 Montgiscard (FR); BOUAYADI, Khalil, F-31520 Ramonville Saint Agne (FR)
(74) Representative: Lavoix
(86) International application number: PCT/EP2012/062273
(87) International publication number: WO 2012/175751

(56) References cited:
- EP-A1- 2 233 500
- WO-A2-2010/106180
- AN ZHIQIANG ET AL: "IgG2m4, an engineered antibody isotype with reduced Fc function.", MABS 2009 NOV-DEC LNKD- PUBMED:20073128, vol. 1, no. 6, November 2009 (2009-11), pages 572-579, XP002665505, ISSN: 1942-0870 cited in the application
- OGANESYAN VAHEH ET AL: "Structural characterization of a human Fc fragment engineered for lack of effector functions", ACTA CRYSTALLOGRAPHICA SECTION D: BIOLOGICAL CRYSTALLOGRAPHY, MUNKSGAARD PUBLISHERS LTD. COPENHAGEN, DK, vol. 64, no. 6, 1 June 2008 (2008-06-01), pages 700-704, XP009108181, ISSN: 0907-4449, DOI: 10.1107/S0907444908007877 cited in the application
- XU DANLIN ET AL: "In vitro characterization of five humanized OKT3 effector function variant antibodies", CELLULAR IMMUNOLOGY, ACADEMIC PRESS, SAN DIEGO, CA, US, vol. 200, no. 1, 25 February 2000 (2000-02-25), pages 16-26, XP002376698, ISSN: 0008-8749, DOI: 10.1006/CIMM.2000.1617 cited in the application
- ABES R ET AL: "Impact of glycosylation on effector functions of therapeutic IgG", PHARMACEUTICALS 2010 MOLECULAR DIVERSITY PRESERVATION INTERNATIONAL CHE LNKD- DOI:10.3390/PH3010146, vol. 3, no. 1, 2010, pages 146-157, XP002665506, ISSN: 1424-8247 cited in the application
- HEZAREH M ET AL: "Effector function activities of a panel of mutants of a broadly neutralizing antibody against human immunodeficiency virus type 1", JOURNAL OF VIROLOGY, THE AMERICAN SOCIETY FOR MICROBIOLOGY, US, vol. 75, no. 24, 1 December 2001 (2001-12-01), pages 12161-12168, XP002590011, ISSN: 0022-538X, DOI: 10.1128/JVI.75.24.12161-12168.2001 cited in the application

## Description

### Field of the Invention

The present invention relates to a method for preparing Fc variants displaying reduced effector activities.

### Description of Related Art

Therapeutics derived from antibodies are more and more used in the treatment of a variety of pathological conditions such as cancers, autoimmune disorders, graft rejections and infectious diseases. In 2010, more than thirty seven antibodies and derived proteins were approved for clinical use and more than 1137 were in clinical development from which 71 % were full IgG. Most of them (91 %) contains Fc region from IgG and corresponds to full-length monoclonal antibodies (mAbs) or fusion proteins also called immunoadhesins (Thompson Pharma August 2010).

Various studies have shown that the effector functions of antibodies are crucial for the efficiency of immunotherapy in particular for the treatment of cancers in which the destruction of the cell target is sought. The effector functions which enable to eradicate cell target mostly encompass antibody-dependent cell-mediated cytotoxicity (ADCC), complement-dependent cytotoxicity (CDC) and antibody-dependent cellular phagocytosis (ADCP). CDC is primarily triggered through the direct binding of Fc domain with the first complement component C1q whereas ADCP and ADCC are triggered by the binding of Fc domain with Fc gamma receptors (FcγRs). Fc domain of antibodies are also involved in serum persistence through interaction with the neonatal Fc receptor (FcRn). Over the past decade, tremendous studies have been thus focused on enhancing the ability of antibodies to induce ADCC and CDC responses and to increase their serum half-life by improving the affinity for FcRn.

However, in the treatment of some specific conditions, the induction of ADCC, CDC and/or ADCP is not required for achieving therapeutic effect. In some cases such inductions have to be avoided in order to reduce side-effects and prevent IgG-cytotoxicity such as in the case of the treatment of ongoing graft rejection with the anti-CD3 monoclonal antibody orthoclone OKT3. The administration of orthoclone OKT3 was shown to induce massive systemic release of pro-inflammatory cytokines which is a consequence of the binding of orthoclone OKT3 to Fc gamma receptors. It has also been shown that activation of FcγRs may have an adverse impact on the treatment such as for the EGFR-targeting antibody, cetuximab, which has been suggested to active tumor-promoting M2 macrophages and decrease progression-free survival of patients (Pander J. et al., 2011).

From a general point of view, when the antibody or the immunoadhesin is only used as a blocking or neutralizing agent directed to an endogenous or infectious target or as an agonist or antagonist of a cell receptor, the recruitment of immune system through the binding of Fc region of said antibody or immunoadhesin to FcγR and C1q is not crucial for the therapeutic efficiency of immunotherapy and even should be thus avoided.

In this respect, it was shown that the four human IgG subclasses exhibit distinct effector functions. On the one hand, IgG1 and IgG3 trigger both ADCC and CDC activities. On the other hand, IgG2 elicits CDC activity but not ADCC and IgG4 displays a very poor ability to induce complement and cell activation because of low affinity for C1q and Fc gamma receptors. Consequently, IgG4 has become the preferred subclass for immunotherapy, in which recruitment of host effector function is undesirable. Neutralizing IgG4 antibodies have been already approved for the treatment of specific conditions. For example, natalizumab is an anti-α4 integrin mAb for the treatment of multiple sclerosis. Despite this fact, some reluctance concerning the use of IgG4 in immunotherapy remains due to its *in vivo* instability and dynamics.

As an alternative, an IgG4 Fc variant comprising the amino acid modification. S228P was conceived. Said modification was shown to stabilize the heavy chain dimer formation with still possible Fab arm exchange in low proportions (<8,3%) (Labrijn et al., Nature Biotech, 2009, 27, 767-771). IgG hybrids were also engineered in order to generate new therapeutic mAbs with low effector activities and improved pharmacological profile (Reddy et al., The journal of Immunology, 2000, 164, 1925-1933). Alexion Pharmaceuticals have developed a humanized IgG2/4 kappa antibody against the complement component C5 named Eculizumab. The heavy chains of Eculizimab are comprised of human IgG2 sequences in constant region 1 (CH1), the hinge and the adjacent portion of constant region 2 (CH2), and human IgG4 sequences in the remaining part of CH2 and constant region 3 (CH3). Eculizimab has been approved for the treatment of paroxysmal nocturnal hemoglobinuria and haemolytic uraemic syndrome by the European Medecines Agency.

Based on the structural differences between IgG2 and IgG4, An *et al*. conceived an IgG2 variant with altered effector functions by introducing four amino acids from IgG4 (namely 268Q/309L/330S/331S) into the corresponding positions in IgG2 backbone. The resulting IgG exhibited no detectable binding to C1q, FcγRI and FcγRIIIa and a poor affinity for FcγRIIb/c while still having a serum half-life similar to that of wild-type IgG2 (An et al., mAbs, 2009,1:6,572-579).

Moreover, various site-specific and random mutagenesis studies in the Fc region of IgG1 had led to the identification of critical amino acids involved in the binding of IgG1 to C1q and to various ADCC-promoting receptors (for review see Strohl, Current opinion in Biotechnol, 2009, 20, 685-691). Amino acids in the lower hinge domain and, in particular, leucine residues at positions 234 and 235 were shown to be critical for the affinity of IgG1 Fc region for both C1q, FcγRI, FcγRII and FcγRIII. Determinant positions were also found in CH2 domain such as amino acid positions 327, 330 and 331 which mutations may greatly reduce the ability to induce both ADCC and CDC responses.

Accordingly, Xu *et al*. showed that the introduction of the mutations 234A/235A in a human IgG1-based OKT3 antibody significantly reduces the binding to FcγRI, FcγRII and C1q which prevents the cytokine release syndrome observed with unmodified OKT3 antibody while maintaining the ability to reverse ongoing graft rejection (Xu et al., Cell Immunol, 2000, 1:16-26).

The same observation concerning the affinity for effector molecules was made by Hezareh *et al*. for an anti-HIV-1 IgG1 variant comprising the mutations 234A/235A (Hezareh et al., Journal of virology, 2001, 12161-12168).

In the same way, Oganesyan *et al*. described that the introduction of the triple mutation 234F/235E/331S in an anti-CD19 IgG1 resulted in a complete loss of the binding to several effector molecules, namely FcγRI, FcγRIIa and FcγRIII and C1q (Oganesyan et al. Acta cryst., 2008, D64, 700-704).

Similarly, InvivoGen marketed a plasmid encoding for a human engineered IgG1 Fc variant comprising the mutations 233P/234V/235A/236DeI/327G/330S/331S. Such mutations drastically reduce the ability of the IgG1 Fc variant to induce ADCC and CDC.

WO2010/106180 describes Fc variants having increased binding affinity for FcRn.

All these inventions described above imply substitution of human amino acids by residues that are unexpected at these positions and could potentially raise immune response when they are used in mAbs that are administered to patients.

It was also shown that the glycosylation on asparagine at position 297 of the Fc region has a direct impact on the ability of monoclonal antibodies to bind FcγR and to trigger ADCC (for review see Abès et al., Pharmaceuticals, 2010, 3, 146-157).

Although Fc variants exhibiting low ADCC and CDC activities were described in the prior art, there is still a need for a method for preparing novel Fc variants exhibiting reduced affinity to C1q and Fc gamma receptors.

### Summary of the invention

The invention relates to a method for producing a variant of a parent polypeptide comprising a Fc region of SEQ ID NO:1, which variant exhibits reduced binding to the protein C1q and to at least one receptor FcγR as compared to the said parent polypeptide, wherein an amino acid modification selected from the group consisting of 294Del, wherein said amino acid modification 294del is the sole amino acid modification introduced in the Fc region of the parent polypeptide, 293Del and 293Del/294Del is introduced within the Fc region of the parent polypeptide, the numbering of the Fc region referring to the numbering according to the EU index (as in Kabat), or its equivalent in Kabat (Kabat numbering).

Another object of the invention is a variant of a parent polypeptide comprising a Fc region of SEQ ID NO:1, which variant exhibits reduced binding to the protein C1q and to at least one receptor FcγR chosen from FcγRII and FcγRIII as compared to the said parent polypeptide and comprises an amino acid modification selected from the group consisting of 293Del/294Del within its Fc region, the numbering of amino acids in the Fc region referring to the numbering according to the EU index, or equivalent in Kabat.

The invention also relates to pharmaceutical composition comprising the said variant, and to the use of the said variant as defined in claim 12 for preventing or treating a pathological condition wherein the induction of ADCC and/or CDC response is not desirable.

### Brief description of the drawings

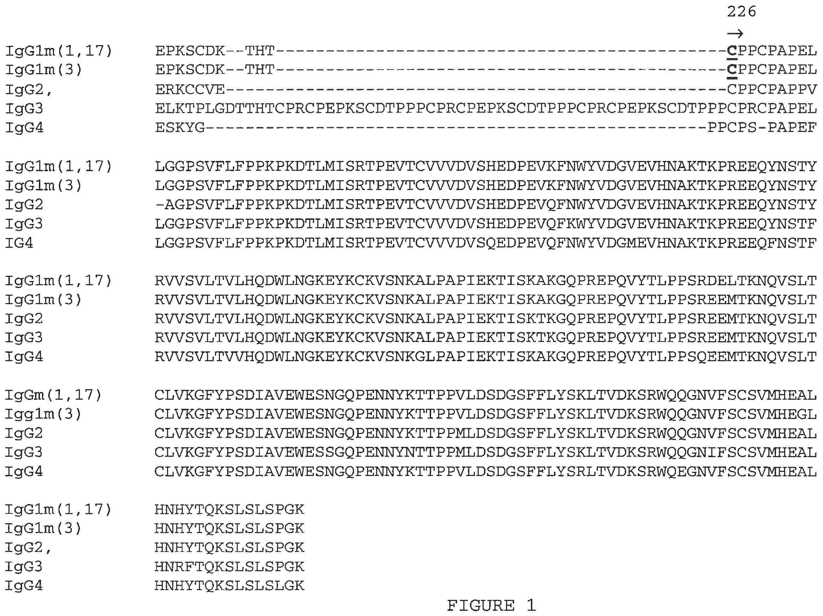

**Figure 1** shows alignments of native human IgG1 sequences referring to positions 216-447 (according to EU index) with the corresponding sequences of human IgG2 (SEQ ID NO:7), human IgG3 (SEQ ID NO:8) and human IgG4 (SEQ ID NO:9). The IgG1 sequences refer to G1m1,17 allotype (SEQ ID NO:5) and to G1m3 allotype (SEQ ID NO:6). The "lower hinge-CH2-CH3" domain of IgG1 begins at position 226 (see arrow).

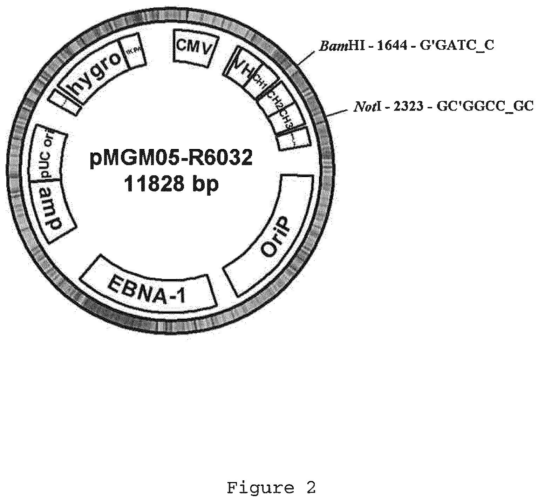

**Figure 2** shows the plasmid vector pMGM05-R603 in which human Fc gene encoding amino acid residues 226-447 (according to EU index) derived from a human IgG1 heavy chain (Fc226, SEQ n°1) was cloned into between the two restriction sites *BamHI* and *Notl.*

### Detailed description of the invention

### a. Definitions

In order that the application may be more completely understood, several definitions are set forth below. Such definitions are meant to encompass grammatical equivalents.

Throughout the present specification and claims, the numbering of the residues in the Fc region is that of the immunoglobulin heavy chain according to the EU index as described in Kabat et al., Sequences of Proteins of Immunological Interest, 5th Ed. Public Health Service, National Institutes of Health, Bethesda, Md. (1991). The "EU index" or "EU index as in Kabat" herein refers to the residue numbering of the human IgG1 EU antibody. The equivalent in Kabat refers to the number in Kabat numbering.

By "polypeptide" or "protein" as used herein is meant at least two covalently attached amino acids, which includes proteins, polypeptides, oligopeptides and peptides.

By "amino acid" as used herein is meant one of the 20 naturally occurring amino acids or any non-natural analogues that may be present at a specific, defined position.

By "amino acid modification" herein is meant a change in the amino acid sequence of a polypeptide. "Amino acid modifications" which may be also termed "amino acid changes", herein include amino acid mutations such as substitution, insertion, and/or deletion in a polypeptide sequence. By "amino acid substitution" or "substitution" herein is meant the replacement of an amino acid at a particular position in a parent polypeptide sequence with another amino acid. For example, the substitution N434S refers to a variant polypeptide, in this case an Fc variant, in which the asparagine at position 434 is replaced with serine. By "amino acid insertion" or "insertion" as used herein is meant the addition of an amino acid at a particular position in a parent polypeptide sequence. For example, insert G>235-236 designates an insertion of glycine between positions 235 and 236. By "amino acid deletion" or "deletion" as used herein is meant the removal of an amino acid at a particular position in a parent polypeptide sequence. E294Del or 294Del designates that the amino acid at position 294 (herein a glutamate) is deleted. An amino acid modification may refer to a punctual mutation or a combination of mutations.

In case of a combination of amino acid mutations, the preferred format is the following: 294Del/2591/315D/434Y or E294Del/V2591/N315D/N434Y. That means that there are four amino acid mutations in the Fc region of the variant as compared to its parent polypeptide: one in position 294, one in position 259, one in position 315 and one in position 434, and that amino acid in position 294, i.e. glutamate, is deleted, the amino acid in position 259 of the parent polypeptide, i.e. valine, is replaced by isoleucine, that the amino acid in position 315 of the parent polypeptide, i.e. asparagine, is replaced by aspartic acid and that the amino acid in position 434 of the parent polypeptide, i.e. asparagine, is replaced by tyrosine. In the same way, the amino acid modification 293Del/294Del means that the amino acids on position 293 and 294 are deleted as compared to the polypeptide parent.

The term "antibody" is used herein in the broadest sense. "Antibody" refers to any polypeptide which at least comprises (i) a Fc region and (ii) a binding polypeptide domain derived from a variable region of an immunoglobulin. Antibodies thus include, but are not limited to, full-length immunoglobulins, multi-specific antibodies, Fc-fusion protein comprising at least one variable region, synthetic antibodies (sometimes referred to herein as "antibody mimetics"), engineered antibodies comprising more than one Fc region, chimeric antibodies, humanized antibodies, fully human antibodies, antibody-fusion proteins, antibody conjugates and fragments of each respectively.

By "full-length antibody" or by "immunoglobulin" as used herein is meant the structure that constitutes the natural biological form of an antibody, including variable and constant regions. "Full length antibody" covers monoclonal full-length antibodies, wild-type full-length antibodies, chimeric full-length antibodies, humanized full-length antibodies, fully human full-length antibodies, the list not being limitative.

In most mammals, including humans and mice, the structure of full-length antibodies is generally a tetramer. Said tetramer is composed of two identical pairs of polypeptide chains, each pair having one "light" chain (typically having a molecular weight of about 25 kDa) and one "heavy" chain (typically having a molecular weight of about 50-70 kDa). In some mammals, for example in camels and llamas, full-length antibodies may consist of only two heavy chains, each heavy chain comprising a variable domain attached to the Fc region.

The amino-terminal portion of each chain includes a variable region of about 100 to 110 or more amino acids primarily responsible for antigen recognition and comprising the so-called complementarity-determining regions (CDR).

The carboxy-terminal portion of each chain defines a constant region primarily responsible for effector functions.

In the case of human immunoglobulins, light chains are classified as kappa and lambda light chains. Heavy chains are classified as mu, delta, gamma, alpha, or epsilon, and define the antibody's isotype as IgM, IgD, IgG, IgA, and IgE, respectively.

By "IgG" as used herein is meant a polypeptide belonging to the class of antibodies that are substantially encoded by a recognized immunoglobulin gamma gene. In humans, IgG comprises the subclasses or isotypes IgG1, IgG2, IgG3, and IgG4. In mice, IgG comprises IgG1, IgG2a, IgG2b, IgG3. Full-length IgGs consist of two identical pairs of two immunoglobulin chains, each pair having one light and one heavy chain, each light chain comprising immunoglobulin domains VL and CL, and each heavy chain comprising immunoglobulin domains VH, Cγ1 (also called CH1), Cγ2 (also called CH2), and Cγ3 (also called CH3). In the context of human IgG1, "CH1" refers to positions 118-215, CH2 domain refers to positions 231-340 and CH3 domain refers to positions 341-447 according to the EU index or respectively 114-223, 244-360 and 361-478 in Kabat numbering. IgG1 also comprises a hinge domain which refers to positions 216-230 in the case of IgG1 according to the EU index or 226-243 in Kabat.

By "Fc" or "Fc region", as used herein is meant the constant region of a full-length immunoglobulin excluding the first constant region immunoglobulin domain. Thus Fc refers to the last two constant region immunoglobulin domains of IgA, IgD, and IgG, the last three constant region immunoglobulin domains of IgE and IgM, and the flexible hinge N-terminal to these domains. For IgA and IgM, Fc may include the J chain. For IgG, Fc comprises immunoglobulin domains CH2, CH3 and the lower hinge region between CH1 and CH2. In other words, Fc region of IgG1 consists of "lower hinge-CH2-CH3" domain i.e the domain from amino acid C226 to the carboxyl-terminus end, wherein the numbering is according to the EU index or equivalent in Kabat . The analogous domains for other IgG sub-classes can be determined from amino acid sequence alignment of heavy chains or heavy chain fragments of said IgG sub-classes with that of human IgG1.

By "Fc polypeptide" as used herein is meant a polypeptide that comprises all or a part of an Fc region. Fc polypeptides include, but are not limited to, antibodies, Fc fusions, isolated Fcs, Fc-conjugates and Fc fragments.

By "parent polypeptide" or "polypeptide parent" as used herein is meant an unmodified polypeptide that is subsequently modified to generate a variant. Said polypeptide may comprise one single polypeptide chain or several polypeptide chains which are not covalently linked together. Said parent polypeptide may be a naturally occurring polypeptide (wild-type polypeptide), a variant or an engineered version of a naturally occurring polypeptide, or a synthetic polypeptide. An engineered or a variant version of a naturally occurring polypeptide is a polypeptide wich is not encoded by a naturally occurring gene. For example, the engineered polypeptide may be a chimeric antibody or a humanized antibody.

Parent polypeptide may refer to the polypeptide itself, or the amino acid sequence that encodes it. In the context of the present invention, the parent polypeptide comprises an Fc region selected from the group of wild-type Fc regions, their fragments and their mutants. Accordingly, the parent polypeptide may optionally comprise pre-existing amino acid modifications in its Fc region (i.e. an Fc mutant) as compared to wild-type Fc regions. Advantageously, the parent polypeptide is an antibody, an immunoglobulin, an Fc fusion polypeptide, an Fc conjugate, this list not being limitative.

By "variant polypeptide", "polypeptide variant" or "variant" as used herein is meant a polypeptide sequence that differs from that of a parent polypeptide sequence by virtue of at least one amino acid modification in the Fc region.

By "wild-type or WT" herein is meant an amino acid sequence or a nucleotide sequence that is found in nature i.e. that is naturally-occurring, including allelic variations. A WT protein, polypeptide, antibody, immunoglobulin, IgG, etc. have an amino acid sequence or a nucleotide sequence that has not been intentionally modified by molecular biological techniques such as mutagenesis. For example, "wild-type Fc regions" include, without being limited to, Fc region of IgG1 having the sequence SEQ ID N°1, Fc region of IgG2 having the sequence SEQ ID N°2, Fc region of IgG3 having the sequence SEQ ID N°3, and Fc region of IgG4 having the sequence SEQ ID N°4.

The terms "Fc receptor" or "FcR" are used to describe a receptor that binds to an Fc region (e.g., the Fc region of an antibody).

The terms 'Fc gamma receptors", "Fey receptors" or "FcγRs" refer to human receptors which bind Fc region of IgG antibodies. As used herein, FcγRs include FcγRI (CD64), FcγRII (CD32), FcγRIII (CD16) subclasses including their allelic variants and alternatively spliced forms of these receptors.

These FcγRs are also defined as either activating receptors (FcγRI, FcγRIIa/c, FcγRIIIa/b) or inhibitory receptor (FcγRIIb) as they elicit or inhibit immune functions.

FcγRI family is composed of three genes (FCGRIA, FCGRIB and FCGRIC) but only the product of FCGRIA has been identified as full length surface receptor. The said product, namely FcγRI, is expressed by dendritic cells (DC), macrophages and also activated neutrophils.

FcγRII family is composed of three genes (FCGR2A, FCGR2B and FCGR2C) which encode the FcγRIIa, FcγRIIb and FcγRIIc proteins. FcγRIIa is expressed on monocytes, certain dendritic cells and neutrophils. FcγRIIc is expressed on natural killer (NK) cells. FcγRIIb is the broadly expressed FcγR. FcγRIIb is virtually present on all leukocytes with exception of NK cells and T cells.

FcγRIII are composed of two genes FCGR3A and FCGR3B which encode FcγRIIIa and FcγRIIIb. The FcγRIIIa protein is expressed as a transmembrane protein on monocytes, tissue specific macrophages, dendritic cells, δ/γT cells, and natural killer cells. FcγRIIIb is a GPI-anchored receptor expressed on the surface of neutrophils and basophils.

Two alleles of the gene encoding FcγRIIa generate 2 variants differing at position 131 (low-responder FcγRIIaR131 and high-responder FcγRIIaH131). Similarly, two alleles of the gene encoding FcγRIIIa generate 2 variants differing at position 158 (low-responder FcγRIIIaF158 and high-responder FcγRIIIaV158).

Noticeably, NK cells, which are believed to be the crucial mediators of antibody-dependent cell-cytotoxicity, only express FcγRIIIa and FcγRIIc and none of the other FcγRs, in particular, the inhibitory FcγRIIb.

Each FcγR protein has differential ligand binding preferences with respect to IgG subclasses and distinct affinities for IgG subclasses.

Activating FcγRs trigger various immune responses such as phagocytosis, respiratory burst and cytokine production (TNF-α, IL-6) by antigen presenting cells (APC), antibody-dependent cellular cytotoxicity (ADCC) and degranulation by neutrophils and NK cells. Activating FcγRs also play an important role in the clearance of immune complex. On the other hand, the inhibitory receptor FcγRIIb is a critical regulatory element in B-cell homeostasis. It controls the threshold and the extent of cell activation.

As used herein, "Antibody-dependent cell-mediated toxicity" or ADCC refers to a mechanism of cell-mediated immunity whereby an effector cell of the immune system actively lyses a target cell that has been bound by specific antibodies. ADCC is mostly mediated by NK cells but also by other immune cells such as neutrophils and eosinophils. Typically, ADCC results from the activation of NK cells. The activation of NK cells involves the binding of their Fc receptors to the Fc region of IgG bound to antigens present on the surface of target cells. Such interactions induce the release by NK cells of cytokines and cytotoxic granules. To assess the capacity of an antibody to induce ADCC, an assay as described in de Romeuf et al. Br J Haematol. 2008 Mar;140(6):635-43, may be performed.

As used herein, C1q is a hexavalent molecule with a molecular weight of approximately 460,000 kDa and a structure likened to a bouquet of tulips in which six collagenous "stalks" are connected to six globular head regions. C1q forms with the two serine proteases, C1r and C1s, the complex C1 which is the first component of the complement cascade pathway.

"Complement-dependent cytotoxicity" or CDC refers to the lysis of a target cell in the presence of complement. Activation of the classical complement pathway is initiated by the binding of C1q to antibodies which are bound to their cognate antigen. To activate the complement cascade, C1q has to bind to at least two molecules of lgG1, IgG2, or IgG3 but only one molecule of IgM. To assess the ability of an antibody to induce CDC, an assay as described in Romeuf et al., Br J Haematol. 2008 Mar;140(6):635-43, may be performed.

Fc gamma receptors and their functions are reviewed in Nimmerjahn and Ravetch, Nature reviews Immunology, 2008, 8, 34-47.

C1q and its function are reviewed e.g. in Kishore et al., Immunopharmacology, 2000, 49:159-170 and Sjöberg et al. Trends Immunol. 2009 30(2):83-90.

By "FcRn" or "neonatal Fc Receptor" as used herein is meant a protein that binds the IgG antibody Fc region and is encoded at least in part by an FCRN gene. As is known in the art, the functional FcRn protein comprises two polypeptides, often referred to as the heavy chain and light chain. The light chain is beta-2-microglobulin and the heavy chain is encoded by the FCRN gene. FcRn or FcRn protein refers to the complex of α-chain with beta-2-microglobulin. In human, the gene coding for FcRn is called FCGRT. FcRn is involved in the transfer of passive humoral immunity for a mother to her fetus and also in the control of the clearance of IgGs.

FcRn and its function is reviewed e.g. in Roopenian, Nature Reviews Immunology, 2007, 7, 715-725.

### b. Method for decreasing Fc binding to C1q and FcγRs

The present invention relates to a method for preparing Fc variants displaying reduced affinity for C1q and for at least one Fcγ receptor as compared to its parent polypeptide.

The Applicant showed that the introduction of a single amino acid mutation in the Fc region of both wild-type and engineered IgGs enable to significantly reduce the binding of said IgGs for both the protein C1q and Fcγ receptors. The said single mutation corresponds to the deletion of the amino acid on position 294 (called hereunder 294Del), the amino acid numbering referring to the numbering according to the EU index or equivalent in Kabat.

More precisely, the Applicant showed that the IgG1 variant obtained by introducing 294Del in the amino acid sequence of an IgG1 comprising wild-type Fc region (i.e. a Fc region having the amino acid sequence of SEQ ID N°1) failed to bind, or display a reduced binding as compared to corresponding wild-type IgG1 variants, to C1q protein, FcγRIIb (also called CD32b), FcγRIIa (also called CD32a), FcγRIIIa (also called CD16a) and FcγRI (also called CD64) through ELISA assay (see III.2.1 and III.2.2 in the example). Noticeably, the introduction of the mutation 294Del also enabled to abrogate or limit the binding affinity to C1q and Feγ receptors in IgG1 polypeptides initially engineered to exhibit increased affinity for FcRn as compared to wild-type IgG1.

For example, the IgG1 variant having the mutations 294Del/256N/378V/383N/434Y as compared to wild-type IgG1 was unable to bind both C1q and FcγR receptors.

On the contrary, its IgG1 parent, which thus bears the mutations 256N/378V/383N/434Y, had increased capacity to bind C1q and FcγRIIIa as compared to wild-type IgG1.

Similar results were obtained for the variant 294Del/2591/315D/434Y as compared to its parent polypeptide comprising the amino acid modification 2591/315D/434Y. The said parent polypeptide has similar ability to bind C1q and FcγRIIIa as compared to IgG1 wild-type.

Similarly, 294Del, 293Del, 293Del/294Del, 294Del/256N/378V/383N/434Y, 294Del/2591/315D/434Y, 294Del/397M, 294Del/302A, 294Del/434S, 294Del/315D, 294Del/230S, 294Del/307A, 294Del/228R, 230S/315D/428U434Y, 294Del/378V and 294Del/434Y variants display a decreased binding to at least one protein selected from C1q and Feγ receptors as compared to their respective parent polypeptide. At the contrary the said parent polypeptides had increased capacity to bind at least one protein selected from C1q and Feγ receptors as compared to wild-type IgG1. The applicant further showed that the IgG1 variant obtained by introducing 294Del in the amino acid sequence of an IgG1 comprising wild-type Fc region may display a similar binding to FcRn as compared to their respective parent IgG1. By similar binding it is intended that the introduction of the 294Del amino acid modification, the said mutation, only results less than 35 % alteration of FcRn binding for the variants as compared to their parent IgG1. By less than 35 %, it is intended, less than 30 %, less than 25 %, less than 20 %, less than 15 %, less than 10 % and less than 5 %.

It is expected that by the decrease of the ability to bind C1q and FcγRs, the introduction of the mutation 294Del would impact the CDC and the ADCC activity of the IgG1 variant, respectively, as compared to the parent IgG1.

The amino acid at position 293 is also a glutamic acid. Its deletion leads to the same nucleotidic and amino acid sequence as the deletion of the glutamic acid at position 294. Therefore, the variants 293Del and 294Del are the same polypeptides. Accordingly, a first object of the present invention is to provide a method for producing a variant of a parent polypeptide comprising a Fc region, which variant exhibits reduced binding to at least one protein selected from C1q and Feγ receptors as compared to the said parent polypeptide, wherein an amino acid modification selected from the group consisting of 294Del or 293Del and 293Del/294Del is introduced within the Fc region of the parent polypeptide, the numbering of the amino acid in the Fc region referring to the numbering according to the EU index, or equivalent in Kabat.

The variant exhibits reduced binding to C1q and to at least one Feγ receptors as compared to the said parent polypeptide

As explained in the above part entitled "definitions", a parent polypeptide refers to a polypeptide comprising an Fc region. Said parent polypeptide may be a naturally occurring polypeptide (wild-type polypeptide), a variant or an engineered version of a naturally occurring polypeptide, or a synthetic polypeptide. Parent polypeptides include, but are not limited to, antibodies, Fc fusion proteins, Fc conjugates, Fc derivated polypeptides, isolated Fc and fragments thereof. For example, the engineered polypeptide may be a chimeric antibody or a humanized antibody.

The Fc region of the parent polypeptide is preferably selected from the group consisting of wild-type Fc regions of human IgG subclasses, fragments and mutants thereof. Herein, Fc region of human IgGs corresponds to the "lower hinge"-CH2-CH3 domain. The "lower hinge"-CH2-CH3 domain of the wild type human IgG1s refers to amino acids from position 226 to position 447 according to the EU indexor equivalent in Kabat . The analogous domains for other human IgG sub-classes can be determined from amino acid sequence alignment of heavy chains of said IgG sub-classes with that of human IgG1 s as shown in Figure 1.

The human IgG subclasses comprise IgG1, IgG2, IgG3, IgG4, including their various allotypes. The sequences of Fc regions of IgG1, IgG2, IgG3 and IgG4 correlate with sequences of SEQ ID N°1, SEQ ID N°2, SEQ ID N°3 and SEQ ID N°4 respectively.

Fragments of Fc region are defined as polypeptides which comprise one or more polypeptides derived from a wild-type Fc region. The said fragment of the Fc region preferably comprises at least 100 consecutive residues from wild-type Fc region. At least 100 consecutive residues from a wild-type Fc region encompasses at least 140, at least 160, at least 200, at least 210 consecutive residue of said wild-type Fc region.

As mentioned above, the parent polypeptide can comprise a wild-type Fc mutant i.e a Fc region which already comprises pre-existing amino acid mutations such as additions, insertions and/or substitutions as compared to wild-type Fc regions.

As previously mentioned, "variant polypeptide" or "variant" as used herein is meant a polypeptide sequence which differs from that of a parent polypeptide in virtue of at least one amino acid modification. In the present case, the at least one amino acid modification necessarily encompasses an amino acid modification selected from the mutation 294Del, the mutation 293Del and the combination of mutation 293Del/294Del.

The variant polypeptide according to the present invention exhibits a reduced binding to the first complement component C1q as compared to its parent polypeptide. In other words, the affinity of the variant for C1q is lower than that of the parent polypeptide.

The variant polypeptide according to the present invention also exhibits an affinity for at least one Feγ receptor lower than that of its parent polypeptide. As used herein, Feγ receptors include FcγRI, FcγRIII and FcγRII receptors. Preferably, the at least one FcγR is selected from the group consisting of FcγRIIIa, FcγRIIa, FcγRI and FcγRIIb.

In some embodiments, the variant polypeptide exhibits a reduced binding to both C1q and FcγRIIIa as compared to its polypeptide parent.

In certain embodiments, the variant polypeptide exhibits a reduced binding to C1q, FcγRIIa and FcγRIIIa as compared to its polypeptide parent.

In other embodiments, the variant polypeptide exhibits a reduced binding to C1q, FcγRIIIa, FcγRIIa and FcγRI as compared to its polypeptide parent.

In other embodiments, the variant polypeptide exhibits a reduced binding to C1q, FcγRIIIa, FcγRIIa, FcγRI and FcγRIIb as compared to its polypeptide parent.

The binding for C1q or for anyone of Fc gamma receptors can be evaluated by well-known methods of the prior art such as ELISA assay, flow cytometry and Surface Plamon Resonance (SPR).

For example, the bond strength of a variant of the invention for a protein of interest (such as C1q or a FcγR) may be compared to that of its parent polypeptide by calculating the ratio of their specific signals obtained by ELISA assay as described in the section III of the example. As used herein, a variant exhibits a reduced binding for a protein of interest such as C1q or FcγR as compared to its parent polypeptide if the ratio obtained by dividing the specific signal of said variant by that of the parent polypeptide is lower than 0.50, the said specific signals being determined by ELISA assay. In other words, the specific signal of the variant is 0.50-fold lower than the specific signal of its parent polypeptide. A ratio lower than 0.50 includes a ratio lower than 0.45, lower than 0.40, lower than 0.35, lower than 0.30, lower than 0.25, lower than 0.20, lower than 0.15, lower than 0.10, lower than 0.05, lower than 0.01.

In a preferred embodiment, the ratio is lower than 0.20.

The preferred format for ELISA assay comprises the coating of the protein of interest.

As an alternative, the binding of the variant and that of its polypeptide parent for a protein of interest may be compared through the determination of EC50 by an appropriate ELISA assay. The EC50 refers to the concentration of the variant which provides a signal representing 50% of the saturation of the curve relating to the percentage of bound protein of interest versus the log of the concentration of the variant. Generally, it is admitted that a variant displays a reduced binding to a protein of interest as compared to its polypeptide parent if its EC50 is at least 1.5-fold higher than that of its polypeptide parent.

The binding affinity of the variant to a protein of interest may also be assessed by SPR through the determination of the constant of dissociation (KD). Generally it is admitted that a variant displays a reduced binding to a protein of interest as compared to its polypeptide parent if its KD is at least 1.5-fold higher than that of its polypeptide parent

The affinity of the variant for C1q or for a FcγR may be so weak that the specific signal by ELISA assay and even the Kd by SPR or the EC50 by ELISA assay cannot be accurately determined since the binding signal is in the background noise or under the threshold of detection. In such a case, the variant is considered not to bind the protein of interest.

For example, the variant obtained by the method according to the invention may not bind to at least one FcγR and exhibits a reduced binding to C1q. Such a variant is clearly illustrated in the examples of the present application.

In some embodiments, the variant of the invention does not bind to at least one protein selected from C1q and Feγ receptors.

The Applicant showed that the introduction of 294Del is sufficient to significantly impair the binding to C1q and to Feγ receptors. In other words, no mutation other than 294Del has to be introduced within the Fc region of the polypeptide parent in order to obtain a variant with appropriate reduced binding to C1q and/or Feγ receptors.

Such a result is particularly surprising since, to the Applicant's knowledge, the prior art mostly describes Fc variants displaying reduced binding to both C1q and Feγ receptors which have at least two amino acid modifications in their Fc region as compared to its polypeptide parent.

In some embodiments, the mutation 294Del is thus the sole amino acid modification introduced in the Fc region of the parent polypeptide to obtain the said variant.

In some other embodiments, the mutation 293Del is the sole amino acid modification introduced in the Fc region of the parent polypeptide to obtain the said variant.

In some further embodiments, the amino acid modification del293/del294 is the sole amino acid modification introduced in the Fc region of the parent polypeptide to obtain the said variant.

Accordingly, certain embodiments of the invention encompass a method for producing a variant of a parent polypeptide comprising a Fc region, which variant exhibits reduced binding to the protein C1q and/or to at least one receptor FcγR as compared to the said parent polypeptide, wherein an amino acid modification selected from the group consisting of:
(i) 294del, wherein said amino acid modification 294del is the sole amino acid modification introduced in the Fc region of the parent polypeptide,
(ii) 293del, and
(iii) 293del/294del,
is introduced within the Fc region of the parent polypeptide, the numbering of amino acids in the Fc region referring to the numbering according to the EU index or equivalent in Kabat.

Other embodiments of the invention encompass a method for producing a variant of a parent polypeptide comprising a Fc region, which variant exhibits reduced binding to the protein C1q and/or to at least one receptor FcγR as compared to the said parent polypeptide, wherein an amino acid modification selected from the group consisting of:
(i) 294del, wherein said amino acid modification 294del is the sole amino acid modification introduced in the Fc region of the parent polypeptide,
(ii) 293del, wherein said amino acid modification 293del is the sole amino acid modification introduced in the Fc region of the parent polypeptide and
(iii) 293del/294del,
is introduced within the Fc region of the parent polypeptide, the numbering of amino acids in the Fc region referring to the numbering according to the EU index,or equivalent in Kabat.

In particular embodiments the method of the invention provides variants which are different from the variant consisting in E294Del/T307P/N434Y

Without to be bound by any theory, the Applicant believes that the method provided by the present invention does not significantly cause major structural rearrangement in the Fc region so that in some cases, the other functions which are not mediated by the binding to C1q and FcγRs are not significantly altered as compared to those of the polypeptide parent. Noticeably, the Applicant showed for various distinct parent polypeptides that the introduction of the mutation 294Del in their Fc region does not significantly impair their affinity for neonatal Fc Receptor (FcRn). For example, the specific signal of the IgG1 variant 256N/294Del/378V/383N/434Y is equal to 0.75-fold that of its polypeptide parent 256N/378V/383N/434Y and the specific signal of the IgG1 variant 307A/294Del is equal to 0.97-fold that of its polypeptide parent 307A. Noticeably, said variants exhibit an increased binding to FcRn as compared to wild-type IgG1 (see table 1-4 in the example). In other words, in some cases, the Fc polypeptide parent and the variant obtained by the method of the present invention may display close binding property for FcRn.

As used herein, the variant obtained by the method of the present invention has a binding to FcRn close to that of its polypeptide parent when the specific signal of the variant is at least equal to 0.6-fold that of its parent polypeptide, the specific signals being determined through an ELISA assay in which FcRn molecules are preferably immobilized as described in the section III of the example. By at least equal to 0.6fold, it is herein intended, 0.6 fold, 0.65 fold, 0.70 fold, 0.75 fold, 0.80 fold, 0.85 fold, 0.90 fold and 0.95 fold.

As mentioned hereabove, the Fc region of the parent polypeptide may be selected from the group consisting of wild-type Fc regions of human IgGs, fragments and mutants thereof.

The wild-type Fc regions of human IgGs encompass polypeptide of SEQ ID N°1, polypeptide of SEQ ID N°2, polypeptide of SEQ ID N°3, polypeptide of SEQ ID N°4, polypeptide of SEQ ID N°5.

In a preferred embodiment, the Fc region of the parent polypeptide is selected from the group consisting of wild-type Fc regions from IgG1 and IgG2, fragments and mutants thereof. In a more preferred embodiment, the Fc region of the polypeptide parent is an Fc region of a wild-type human IgG1, fragments and mutants thereof.

As indicated hereabove, the Fc region of the parent polypeptide may comprise pre-existing amino acid modifications selecting from deletion, insertion and substitution of one or more amino acids. In other words, the Fc region of the parent polypeptide may be a mutant of a wild-type Fc regions, preferably a mutant of a wild-type Fc region of human IgGs, namely lgG1, IgG2, IgG3 and IgG4.

In some embodiment, the Fc region of the polypeptide parent comprises from about 1 to about 20 amino acid mutations, preferably from about 1 to about 10 amino acid mutations as compared to its corresponding wild-type Fc region.

From about 1 to about 20 amino acid mutations encompasses 1, 2, 3, 4, 5, 6, 7, 8, 9, 10, 11, 12, 13, 14, 15, 16, 17, 18, 19 and 20 amino acid mutations.

The Fc region of the polypeptide parent has generally at least about 90% amino acid identity with its corresponding wild-type Fc region. At least 90% of amino acid identity encompasses at least about 90%, 91%, 92%, 93%, 94%, 95%, 96%, 97%, 98%, 99% and 99.5%.

To determine the percentage of identity between a first polypeptide (A) with a second polypeptide (B), their sequences are aligned by using well-known methods of the prior art such as the global alignment algorithm of Needleman-Wunsch taking into account the eventual gaps (i.e. the eventual deletions and insertions present in the sequence of polypeptide (A) as compared to polypeptide (B)). One may use the alignment tool « EMBOSS Pairwise Alignment Algorithms » available on EMBL-EBI website (www.ebi.ac.uk/Tools/emboss/align/) with the following parameters: (i) Method: EMBOSS: Needle (global); (ii) Gap extend: 0.5; (iii) Gap open: 10.0; (iv) Molecule: Protein; (v) Matrix: Blosum62.

Once the global alignment is obtained, the percentage of identity may be determined by conventional methods, preferably by dividing the number of matches residues resulting from the alignment of (A) and (B) by the number of amino acids in the sequence of the longest polypeptide between (A) and (B).

When the parent polypeptide comprises pre-existing amino acid mutations, the said parent polypeptide may display one or more decreased or increased effector functions as compared to its reference polypeptide i.e. a similar polypeptide comprising wild-type Fc region. Effector functions as used herein include but are not limited to ADCC, ADCP, CDC and binding to FcRn.

The one skilled in the art may refer to previous studies so as to determine the pre-existing amino acid mutations which may be present in the parent polypeptide depending on the effector function profile which is sought. For example, the parent polypeptide may comprise at least one amino acid mutation at an amino position selected from 227, 228, 230, 231, 233, 234, 239, 241, 243, 246, 250, 252, 256, 259, 264, 265, 267, 269, 270, 276, 284, 285, 288, 289, 301, 302, 303, 305, 308, 309, 311, 315,317, 320, 322, 325, 327, 330, 332, 334, 335, 338, 340, 342, 343, 345, 347, 350, 352, 354, 355, 356, 359, 360, 361, 362, 369, 370, 371, 375, 378, 380, 382, 383, 384, 385, 386, 387, 389, 390, 392, 393, 394, 395, 396, 397, 398, 399, 400, 401, 403, 404, 408, 411, 412, 414, 415, 416, 418, 419, 420, 421, 422, 424, 426, 428, 433, 438, 439, 440, 443, 444, 445, 446 and 447 of the Fc region wherein the numbering of the amino acids in the Fc region is that of the EU indexor equivalent in Kabat.

The parent polypeptide may also comprise only one amino acid mutation at an amino acid position selected from 227, 228, 230, 231, 233, 234, 239, 241, 243, 246, 250, 252, 256, 259, 264, 265, 267, 269, 270, 276, 284, 285, 288, 289, 301, 302, 303, 305, 307, 308, 309, 311, 315,317, 320, 322, 325, 327, 330, 332, 334, 335, 338, 340, 342, 343, 345, 347, 350, 352, 354, 355, 356, 359, 360, 361, 362, 369, 370, 371, 375, 378, 380, 382, 383, 384, 385, 386, 387, 389, 390, 392, 393, 394, 395, 396, 397, 398, 399, 400, 401, 403, 404, 408, 411, 412, 414, 415, 416, 418, 419, 420, 421, 422, 424, 426, 428, 433, 434, 438, 439, 440, 443, 444, 445, 446 and 447 of the Fc region wherein the numbering of the amino acids in the Fc region is that of the EU index, or equivalent in Kabat. "Only one amino acid mutation" means that the parent polypeptide does not contain more than one modification on the positions cited hereabove. For example, the parent polypeptide does not comprise amino acid modifications on both positions 307 and 434 of the Fc region. Accordingly, in the embodiment of the method wherein the sole mutation introduced within the Fc region of the polypeptide parent is 294Del or 293Del, the resulting variant cannot comprise more than one amino acid mutation on amino acid positions cited hereabove, in particular the variant does not comprise amino acid mutations on both positions 307 and 434 of the Fc region, the numbering of the amino acids in the Fc region is that of the EU index, or equivalent in Kabat.

Accordingly certain embodiments of the invention encompass a method for producing a variant of a parent polypeptide comprising a Fc region, which variant exhibits reduced binding to at least one protein selected from C1q and Fcγ receptors as compared to the said parent polypeptide, wherein an amino acid modification selected from the group consisting of 294Del or 293Del and 293Del/294Del is introduced within the Fc region of the parent polypeptide, with the proviso the resulting variant does not comprise the 294Del/T307P/N434Y or the 293Del/T307P/N434Y mutations.

In other embodiments, the invention encompass a method for producing a variant of a parent polypeptide comprising a Fc region, which variant exhibits reduced binding to at least one protein selected from C1q and Feγ receptors as compared to the said parent polypeptide, wherein an amino acid modification selected from the group consisting of 294Del or 293Del and 293Del/294Del is introduced within the Fc region of the parent polypeptide, with the proviso the resulting variant does not consist in the 294Del/T307P/N434Y or the 293Del/T307P/N434Y variants.

Other embodiments of the invention encompass a method for producing a variant of a parent polypeptide comprising a Fc region, which variant exhibits reduced binding to at least one protein selected from C1q and Fcγ receptors as compared to the said parent polypeptide, wherein an amino acid modification selected from the group consisting of 294Del or 293Del and 293Del/294Del is introduced within the Fc region of the parent polypeptide, with the proviso that said variant of the parent polypeptide does not comprise an amino acid modification consisting in 294Del or 293Del combined to mutations in both positions 307 and 434, the numbering of the amino acid in the Fc region referring to the numbering according to the EU index or equivalent in Kabat. In the same way, the polypeptide parent may or may not comprise at least one amino acid modification at positions from 290 to 292 and from 295 to 300 of the Fc region wherein the numbering of the amino acids in the Fc region is that of the EU index or equivalent in Kabat.

Accordingly, in some embodiments, the polypeptide parent does not comprise amino acid modifications at anyone of amino acid positions from 290 to 292 and from 295 to 300 of the Fc region wherein the numbering of the amino acids in the Fc region is that of the EU index, or equivalent in Kabat.

The amino acid positions from 290 to 292 and from 295 to 300 encompass 290, 291, 292, 295, 296, 297, 298, 299 and 300.

In other embodiments, the polypeptide parent does not comprise any mutation in its Fc region as compared to wild-type Fc regions. In such embodiments, the parent polypeptide comprises a Fc region selected from the group consisting of wild-type Fc regions of human IgGs and fragments thereof.

For reminder, wild-type Fc regions of human IgGs encompass the Fc region of SEQ ID N°1, the Fc region of SEQ ID N°2, the Fc region of SEQ ID N°3 and the Fc region of SEQ ID N°4.

In some other embodiments, the Fc region of the parent polypeptide is a variant of a wild-type IgG Fc region comprising at least an amino acid modification selected from 434Y, 378V, 397M, 302A, 434S, 315D, 230S, 307A, 228R, 230S/315D/428L/434Y, ,2591/315D/434Y and 256N/378V/383N/434Y.

In certain embodiments, the Fc region of the parent polypeptide is a variant of a wild-type IgG Fc region selected from the group consisting of 434Y, 378V, 397M, 302A, 434S, 315D, 230S, 307A, 228R, 230S/315D/428L/434Y, 2591/315D/434Y and 256N/378V/383N/434Y.

In a specific embodiment, the method for producing a variant of a parent polypeptide comprising a Fc region, which variant exhibits reduced binding to the protein C1q and to at least one receptor FcγR as compared to the said parent polypeptide is characterized in that:
(i) an amino acid modification selected from 294Del, 293Del and 293Del/294Del is introduced within the Fc region of the parent polypeptide,
(ii) the amino acid modification selected from 294Del, 293Del and 293Del/294Del is the sole amino acid modification introduced in the Fc region of the parent polypeptide to obtain the said variant, and
(iii) the Fc region of the parent polypeptide is selected from the group of wild-type Fc regions of IgGs and fragments thereof;
the numbering of amino acids in the Fc region referring to the numbering according to the EU index or equivalent in Kabat.

In some embodiment, the step of introducing an amino acid modification selected from 294Del, 293Del and 293Del/294Del within the Fc region of the parent polypeptide comprising the steps of:
(a) providing a nucleic acid encoding the parent polypeptide,
(b) modifying the nucleic acid provided in step (i) so as to obtain a nucleic acid encoding the said variant, and
(c) expressing the nucleic acid obtained in step (ii) in a host cell and recovering the said variant.

In some embodiment of the method according to the invention, the amino acid modification introduced in the Fc region of the parent polypeptide is 294Del. In other embodiments, the said modification is 293Del.

Such a method may be performed by conventional practices of molecular biology. For carrying out the method of the invention, the one skilled in the art may refer to well-known procedures described in the prior art which may be found e.g. in Molecular Cloning - A Laboratory Manual, 3rd Ed. (Maniatis, Cold Spring Harbor Laboratory Press, New York, 2001), The condensed protocols from Molecular cloning: a laboratory manual (Sambrook, Russell, CSHL Press, 2006), and Current Protocols in Molecular Biology (John Wiley & Sons, 2004).

The nucleic acid of the parent polypeptide may be commercial or may be obtained by classical procedure of molecular biology or chemical synthesis. The nucleic acid encoding the variant as mentioned in step (b) may be achieved by modifying the nucleic acid of the parent polypeptide using a variety of methods known in the prior art. These methods include, but are not limited to site-directed mutagenesis, random mutagenesis, PCR mutagenesis and cassette mutagenesis.

The nucleic acid encoding the said variant may be incorporated into an expression vector in view of its expression in a host cell.

Expression vectors typically include a protein operably linked, that is, placed in a functional relationship, with control or regulatory sequences, selectable markers, any fusion partners, and/or additional elements. The variant of the present invention may be produced by culturing a host cell transformed with nucleic acid, preferably an expression vector, containing nucleic acid encoding the variant, under the appropriate conditions to induce or cause expression of the protein. A wide variety of appropriate host cell lines may be used, including but not limited to mammalian cells, plant cells, bacteria, insect cells, and yeast. In vitro synthesis may also be achieved in cell-free translation systems including but not limited to extracts from rabbit reticulocytes, wheat germ and *Escherichia coli.*

For example, a variety of mammalian cell lines that may find use are described in the ATCC cell line catalogue, available from the American Type Culture Collection. Host cells may be, but not limited to, YB2/0 (YB2/3HL.P2.GII.IGAg.20 cell, deposit to the American Type Culture Collection, ATCC n°CRL-1662), SP2/0, YE2/0, 1R983F, Namalwa, PERC6, CHO cell lines, particularly CHO-K-1, CHO-LecI0, CHO-Lecl, CHO-Lecl3, CHO Pro-5, CHO dhfr-, Wil-2, Jurkat, Vero, Molt-4, COS-7, 293-HEK, BHK, KGH6, NSO, SP2/0-Ag 14, P3X63Ag8.653, C127, JC, LA7, ZR-45-30, hTERT, NM2C5, UACC-812 and the like. The methods of introducing exogenous nucleic acid into host cells are well known in the art, and will vary with the host cell used.

The host cell may belong to a transgenic non-human animal or to a transgenic plant per se. In this case, the variant is thus obtained from a transgenic organism.

A transgenic non-human animal can be obtained by directly injecting a desired gene into a fertilized egg (Gordon et al., 1980 Proc Natl Acad Sci U S A.;77:7380-4). The transgenic non-human animals include mouse, rabbit, rat, goat, cow, cattle or fowl, and the like. A transgenic non-human animal having a desired gene can be obtained by introducing the desired gene into an embryonic stem cell and preparing the animal by an aggregation chimera method or injection chimera method (Manipulating the Mouse Embryo, A Laboratory Manual, Second edition, Cold Spring Harbor Laboratory Press (1994); Gene Targeting, A Practical Approach, IRL Press at Oxford University Press (1993)). Examples of the embryonic stem cell include embryonic stem cells of mouse (Evans and Kaufman, 1981, Nature; 292:154-156), rat, goat, rabbit, monkey, fowl, cattle and the like. In addition, a transgenic non-human animal can also be prepared using a clonal technique in which a nucleus into which a desired gene is introduced is transplanted into an enucleated egg (Ryan et al., 1997 Science; 278: 873 - 876 ; Cibelli et al., 1998 Science, 280 : 1256-1258). The polypeptide variant can be produced by introducing DNA encoding the variant molecule into an animal prepared by the above method to thereby form and accumulate the variant molecule in the animal, and then collecting the polypeptide variant from the animal. The polypeptide variant may be made to be formed and accumulated in the milk, egg or the like of the animal.

In all the above cited embodiments, the parent polypeptide may be a naturally occurring polypeptide (wild-type polypeptide), a variant or an engineered version of a naturally occurring polypeptide, or a synthetic polypeptide.

In some embodiments, the parent polypeptide is selected from the group consisting of Fc-fusion protein, Fc-conjugate and antibodies.

As used herein, Fc-fusion protein and Fc-conjugate consist of an Fc region linked to a partner. The Fc region can be linked to its partner with or without a spacer.

According to the present invention, an Fc fusion protein is a protein encoded by a single gene and comprises a protein, a polypeptide or a small peptide linked to an Fc region. An Fc fusion protein optionally comprises a peptide spacer. Virtually any protein or small peptide may be linked to Fc regions to generate an Fc fusion. Protein fusion partners may include, but are not limited to, the target-binding region of a receptor, an adhesion molecule, a ligand, an enzyme, a cytokine, a chemokine, or some other protein or protein domain.

In particular the Fc-fusion protein can be an immunoadhesin i.e antibody-like protein which combines the binding domain of a heterologous "adhesion" protein (i.e receptor, ligand or enzyme) with a fragment of immunoglobulin constant domain (i.e. an Fc region) (see for a review about immunoadhesins, Ashkenazi A, Chamow SM. 1997, Curr Opin Immunol. ;9(2):195-200).

Small peptide may include, but are not limited to, any therapeutic agent that directs the Fc fusion to a therapeutic target.

According to the present invention, an Fc conjugate results from the chemical coupling of an Fc region with a conjugate partner and optionally comprises a spacer linking the Fc region to the conjugate partner. The conjugate partner can be proteinaceous or non-proteinaceous. The coupling reaction generally uses functional groups on the Fc region and on the conjugate partner.

Suitable conjugate partners include, but are not limited to, therapeutic polypeptides, labels (for example of labels, see further below), drugs, cytotoxic agents, cytotoxic drugs (e.g., chemotherapeutic agents), toxins and active fragments of such toxins. Suitable toxins and their corresponding fragments include, but are not limited to, diptheria A chain, exotoxin A chain, ricin A chain, abrin A chain, and the like. A cytotoxic agent may be any radionuclide which can be directly conjugated to the Fc variant or sequestrated by a chelating agent which is covalently attached to the Fc variant. In additional embodiments, the conjugate partners can be selected from the group consisting of calicheamicin, auristatins, geldanamycin, maytansine, and duocarmycins and analogs.

As mentioned, the term "antibody" is used herein in the broadest sense. According to the present invention, "antibody" refers to any polypeptide which at least comprises (i) a Fc region and (ii) a binding polypeptide domain derived from a variable domain of an immunoglobulin. The said binding polypeptide domain is able to bind specifically one given target antigen or a group of target antigens. A binding polypeptide domain which derives from a variable region of an immunoglobulin comprises at least one or more CDRs. Herein, antibodies include, but are not limited to, full-length antibodies, multi-specific antibodies, Fc-fusion protein comprising at least one variable region or synthetic antibodies (sometimes referred to herein as "antibody mimetics"), antibody-fusion proteins, antibody conjugates and fragments of each respectively.

By Fc-fusion protein comprising at least one variable region is meant an engineered protein comprising (i) an Fc region and (ii) a binding polypeptide domain derived from a variable domain of an immunoglobulin. Of particular interest are antibodies that comprise (a) a Fc variant of the inventions, and (b) one of the following binding polypeptide domains derived from a variable region of an immunoglobulin (i.e. which comprise at least one CDR) : (i) the Fab fragment consisting of VL, VH, CL and CH1 domains, (ii) the Fd fragment consisting of the VH and CH1 domains, (iii) the Fv fragment consisting of the VL and VH domains of a single antibody; (iv) isolated CDR regions, (v) F(ab')2 fragments, a bivalent fragment comprising two linked Fab fragments (vi) single chain Fv molecules (scFv), wherein a VH domain and a VL domain are linked by a peptide linker which allows the two domains to associate to form an antigen binding site, (vii) bispecific single chain Fv and (viii) "diabodies" or "triabodies", multivalent or multispecific fragments constructed by gene fusion, this list not being limitative.

By "full length antibody" herein is meant an antibody having the natural-occurring biological form of an antibody, including variable and constant regions. A full-length antibody may be a wild-type antibody, a mutant of a wild-type antibody (e.g. comprising pre-existing modifications), an engineered version of a wild-type antibody (e.g. for example a chimeric, a humanized antibody or a fully human antibody, see further below), this list not being limitative. As well-known, the structure of a full-length antibody is generally a tetramer except for some mammals such as llamas and camels in which some immunoglobulins are dimers.

The scaffold components of the full-length antibody may be a mixture from different species. Such antibody variant may be a chimeric antibody and/or a humanized antibody. In general, both "chimeric antibodies" and "humanized antibodies" refer to antibodies that combine regions from more than one species. For example, "chimeric antibodies" traditionally comprise variable region(s) from a non-human animal, generally the mouse (or rat, in some cases) and the constant region(s) from a human. For the most part, humanized antibodies are chimeric antibodies that contain minimal sequence derived from non human immunoglobulin. Generally, in a humanized antibody, the entire antibody, except the CDRs, is encoded by a polynucleotide of human origin or is identical to a human antibody except within its CDRs. The CDRs, some or all of which are encoded by nucleic acids originating in a non-human organism, are grafted into the beta-sheet framework of a human antibody variable region to create an antibody, the specificity of which is determined by the engrafted CDRs. The method for preparing such antibodies are well-known and are described in, e.g., WO 92/11018; Jones, 1986, Nature 321:522-525 ; Verhoeyen et al., 1988, Science 239:1534-1536, Tsurushita & Vasquez, 2004, Humanization of Monoclonal Antibodies, Molecular Biology of B Cells, 533-545, Elsevier Science (USA)).

As used herein, "fully human antibody" or "complete human antibody" refers to an antibody entirely comprising sequences originating from human genes. In some cases this may be human antibodies that have the gene sequence of an antibody derived from a human chromosome with the modifications outlined herein. Alternatively, the components of the antibody may be human but not be derived from a single gene. Thus, for example, human CDRs from one antibody can be combined with sequences, such as scaffold sequences, from one or more human antibodies. For example, a variety of germline sequences can be combined to form a human antibody or human scaffold.

Full-length antibodies comprising covalent modifications are also included within the scope of this invention. Such modifications include, but are not limited to, glycosylations, labelling and conjugation.

Labelling refers to the coupling of a detectable label with the full-length antibody. As use herein, a label includes, without being limited to, : a) isotopic labels, which may be radioactive or heavy isotopes; b) magnetic labels (e.g., magnetic particles); c) redox active moieties; d) optical dyes such as chromophores, phosphors and fluorophores; enzymatic groups (e.g. horseradish peroxidase, β-galactosidase, luciferase, alkaline phosphatase); e) biotinylated groups; and f) predetermined polypeptide epitopes recognized by a secondary reporter (e.g., leucine zipper pair sequences, binding sites for secondary antibodies, metal binding domains, epitope tags, etc.).

Conjugation refers to the coupling of the full-length antibody with a polypeptide or a non-peptide molecule such as a target-binding region of a receptor, an adhesion molecule, a ligand, an enzyme, a cytokine, a chemokine, a drug, a cytotoxic agent (e.g., chemotherapeutic agents) or a toxin.

In certain embodiment, the polypeptide parent is selected from the group consisting of chimeric immunoglobulins, humanized immunoglobulins, fully-human immunoglobulins, immunoglobulins being preferably selected among IgGs and optionally conjugated or labelled.

### c. Variants of the invention

Another object of the present invention is a variant of a parent polypeptide comprising an Fc region, exhibiting reduced binding to at least one protein selected from the protein C1q and to at least one receptor FcγR chosen from FcγRII and FcγRIII as compared to the said parent polypeptide and comprising an amino acid modification consisting of 293Del/294Del within its Fc region, as compared to the Fc region of its polypeptide parent. Said variant may be obtainable by the method of the invention.

The said variant exhibits reduced binding to the protein C1q and to at least one receptor FcγR chosen from FcγRII and FcγRIII as compared to the said parent polypeptide and comprises an amino acid modification consisting of 293Del/294Del within its Fc region, as compared to the Fc region of its polypeptide parent.

The said variant exhibits reduced binding to C1q and at least one receptor Fcγ as compared to the said parent polypeptide.

In some embodiments, the mutation 294Del is the sole mutation that contains the Fc region of the variant as compared to the Fc region of the parent polypeptide.

In other embodiments, the mutation 293Del is the sole mutation that contains the Fc region of the variant as compared to the Fc region of the parent polypeptide.

In some other embodiments, the amino acid modification 293Del/294Del is the sole modification that contains the Fc region of the variant as compared to that of the parent polypeptide.

The structural and the functional properties of the said variants directly flow from the features of the method used for preparing it, the said method being fully-described in part b. entitled "Method for decreasing Fc binding to C1q and FcγRs".

It should be underlined that the properties of the variant can be generally deduced from those of the parent polypeptide except in terms of binding to C1q and Feγ receptors since the binding of the variant to C1q and FcγRs are controlled by the amino acid modification at position 294 and/or at position 293.

Accordingly, in some embodiments, the variant polypeptide exhibits a reduced binding to C1q and to at least one Feγ receptors selected from FcγRIIIa, FcγRIIa, FcγRI and FcγRIIb, as compared to its polypeptide parent.

As used herein, a variant exhibits a reduced binding for a protein of interest such as C1q or FcγR as compared to its parent polypeptide if the ratio obtained by dividing the specific signal of said variant by that of the parent polypeptide is lower than 0.5, the said specific signals being determined by ELISA assay. In other words, the specific signal of the variant is at most equal to 0.5-fold the specific signal of its parent polypeptide.

The Fc region of the variant may comprise one or more amino acid modifications other than 294Del and 293Del as compared to wild-type Fc regions. Generally, the Fc region of the variant comprises from about 1 to about 21 amino acid modifications, preferably from about 1 to about 11 amino acid modifications as compared to its corresponding wild-type Fc region, the said modification including 294Del, 293Del or 293Del/294Del.

Similarly to the parent polypeptide, the variant may further comprise at least one amino acid modification at an amino position selected from 227, 228, 230, 231, 233, 234, 239, 241, 243, 246, 250, 252, 256, 259, 264, 265, 267, 269, 270, 276, 284, 285, 288, 289, 301, 302, 303, 305, 308, 309, 311, 315,317, 320, 322, 325, 327, 330, 332, 334, 335, 338, 340, 342, 343, 345, 347, 350, 352, 354, 355, 356, 359, 360, 361, 362, 369, 370, 371, 375, 378, 380, 382, 383, 384, 385, 386, 387, 389, 390, 392, 393, 394, 395, 396, 397, 398, 399, 400, 401, 403, 404, 408, 411, 412, 414, 415, 416, 418, 419, 420, 421, 422, 424, 426, 428, 433, 438, 439, 440, 443, 444, 445, 446 and 447 of the Fc region as compared to wild-type Fc regions wherein the numbering of the amino acids in the Fc region is that of the EU index, or equivalent in Kabat.

The variant may also comprise only one amino acid modification at an amino acid position selected from 227, 228, 230, 231, 233, 234, 239, 241, 243, 246, 250, 252, 256, 259, 264, 265, 267, 269, 270, 276, 284, 285, 288, 289, 301, 302, 303, 305, 307, 308, 309, 311, 315,317, 320, 322, 325, 327, 330, 332, 334, 335, 338, 340, 342, 343, 345, 347, 350, 352, 354, 355, 356, 359, 360, 361, 362, 369, 370, 371, 375, 378, 380, 382, 383, 384, 385, 386, 387, 389, 390, 392, 393, 394, 395, 396, 397, 398, 399, 400, 401, 403, 404, 408, 411, 412, 414, 415, 416, 418, 419, 420, 421, 422, 424, 426, 428, 433, 434, 438, 439, 440, 443, 444, 445, 446 and 447 of the Fc region as compared to wild-type Fc regions wherein the numbering of the amino acids in the Fc region is that of the EU index, or equivalent in Kabat. "Only one amino acid modification" means that the variant does not contain more than one modification on the positions cited hereabove. For example, the variant does not comprise amino acid modifications on both positions 307 and 434 of the Fc region, as compared to wild-type Fc regions. Accordingly, in certain embodiments of the invention the variant does not comprise the amino acid modification consisting in 294Del/T307P/N434Y or 293Del/T307P/N434Y. In particular embodiments of the invention the variant does not consist in the 294Del/T307P/N434Y or 293Del/T307P/N434Y variants.

In other embodiments of the invention, the variant does not comprise an amino acid modification consisting in 294del or 293del combined to mutations in both positions 307 and 434. Therefore an embodiment of the invention consists in a variant of a parent polypeptide comprising an Fc region, to the protein C1q and to at least one receptor FcγR as compared to the said parent polypeptide and comprising an amino acid modification selected from 294Del, 293Del and 293Del/294Del within its Fc region, as compared to the Fc region of its polypeptide parent, with the proviso that said variant of the parent polypeptide does not comprise an amino acid modification consisting in 294del or 293del combined to mutations in both positions 307 and 434.

In some embodiments, the variant of the invention further comprises at least an amino acid modification selected from 378V, 434Y, 397M, 302A, 434S, 315D, 230S, 307A, 228R, 230S/315D/428L/434Y, 2591/315D/434Y and the amino acid modification 256N/378V/383N/434Y in the Fc region.

In some specific embodiments, the Fc region of the variant of the invention is a variant of a wild-type IgG Fc region comprising at least one mutation selected from the group consisting of 294Del, 293Del, 293Del/294Del, 294Del/256N/378V/383N/434Y, 294Del/2591/315D/434Y, 294Del/397M, 294Del/302A, 294Del/434S, 294Del/315D, 294Del/230S, 294Del/307A, 294Del/228R, 230S/315D/428L/434Y, 294Del/378V and 294Del/434Y of Fc region wherein the numbering of the amino acids in the Fc region is that of the EU index, or equivalent in Kabat.

In the same way, the variant may or may not comprise one amino acid modification at anyone of positions from 290 to 292 and from 295 to 300 of the Fc region as compared to wild-type Fc regions wherein the numbering of the amino acids in the Fc region is that of the EU index, or its equivalent in Kabat.

In certain embodiments, the variant does not comprise any mutation other than 294Del or 293Del in its Fc region which may decrease the binding to C1q and to at least one FcγR as compared to wild-type Fc regions.

In other embodiments, the variant does not comprise any mutation other than 294Del or 293Del in its Fc region as compared to wild-type Fc regions.

In some embodiments, the Fc region of the variant is derived from wild-type Fc regions of IgGs.

In some other embodiments, the Fc region of the variant is selected from the group of variants of IgG wild-type Fc regions consisting of 293Del, 294Del, 293Del/294Del, 378V/294Del, 434Y/294Del, 294Del/397M, 294Del/302A, 294Del/434S, 294Del/315D, 294Del/230S, 294Del/307A, 294Del/228R, 230S/315D/428L/434Y, 2591/315D/434Y/294Del and 256N/378V/383N/434Y/294Del, wherein the numbering of the amino acids refers to the Fc amino acid numbering of the EU index, or equivalent in Kabat.

In other embodiments, the variant is selected from the group consisting of Fc-fusion proteins, Fc-conjugates and antibodies.

In some embodiments, the variant is an antibody selected from the group consisting of chimeric immunoglobulins, humanized immunoglobulins and fully-human immunoglobulins, immunoglobulins being preferably selected among IgGs.

A further object of the invention is an isolated nucleic acid encoding a variant as defined hereabove. The invention also relates to a vector comprising a nucleic acid encoding the said variant and to a host cell comprising the said vector. In a preferred embodiment, the nucleic acid encoding the said vector has been stably integrated in the genome of the host cell. The invention also relates to a non-human transgenic animal comprising the said nucleic acid or the said vector stably integrated within its genome.

### d. Uses of the method and the variants according to the invention

The Applicant showed that the mutation 294Del of the Fc region drastically impairs the affinity of the Fc variant for C1q and for Fc gamma receptors such as FcγRI, FcγRIIa, FcγRIIb and FcγRIIIa. The decrease in the affinity for these effector molecules is so important that in some cases, the binding of the Fc variant to C1q and/or to certain FcγRs cannot be observed *in vitro* by conventional ELISA assay. The binding of the Fc region to C1q is essential for the induction of CDC *in vivo.* In the same way, the binding of the Fc region to FcyRIIa and FcγRIIIa is a key step for the induction of ADCC and ADCP *in vivo.*

Consequently, due to their poor affinity for C1q, the variants of the invention are anticipated to have no CDC activity or to induce a significantly lower CDC response *in vivo* as compared to their parent polypeptides and generally to polypeptides comprising a Fc region without the mutation 294Del or the mutation 293Del. In the same way, due to their poor affinity for certain FcγRs (in particular FcγRIIa and FcγRIIIa), the variants of the invention are anticipated to have no ADCC activity or to induce a significantly lower ADCC response *in vivo* as compared to their parent polypeptides and generally to polypeptides comprising Fc region without the mutation 294Del or the mutation 293Del. The same result is also expected for *in vitro* CDC and ADCC assays.

Indeed it has been shown by the applicant that the IgG1 variant obtained by introducing 294Del in the amino acid sequence of an IgG1 comprising wild-type Fc region (i.e. a Fc region having the amino acid sequence of SEQ ID N°1) display no, or reduced ADCC and/or CDC activity.

Noticeably, the introduction of the mutation 294Del also enabled to limit or abrogate ADCC and/or CDC activities, while preferentially preserving affinity for FcRn in IgG1 polypeptides initially engineered to exhibit increased affinity for FcRn as compared to wild-type IgG1.

For example, the IgG1 variant having the mutations 294Del/256N/378V/383N/434Y displayed neither ADCC nor CDC activity but display an affinity for FcRn at least similar to its IgG1 parent (see ELISA and SPR results in sections III and IV of the example).

Similar results were obtained for the variant 294Del/2591/315D/434Y as compared to its parent polypeptide comprising the amino acid modification 2591/315D/434Y.

Due to their effector activity profiles, the variants of the invention may find use in a wide range of scientific fields. In particular, the variants of the invention may be used as research reagents, diagnostic agents or therapeutics.

For example, the variants may be labelled with a fluorophore or with an isotope such as indium-111 or technetium-99m and be used for *in vivo* imagery since in such an application, the activation of ADCC or CDC is not required.

When used as therapeutics, the variant may be used to convey a therapeutic agent such as radionuclides, toxins, cytokines or enzymes to a target cell for example a cancerous cell. In this case, the variant may be a conjugate between an antibody and the cytotoxic agent and its therapeutic activity relies on the cytotoxic agent (e.g. Gilliland et al., PNAS, 1980, 77, 4539-4543).

The polypeptide variants may also function as a blocking or neutralizing agents of a target molecule, but not killing of the cells bearing target antigen. It may also agonize, antagonize or inhibit a target molecule.

In these cases depletion of target cells is undesirable and can be considered a side effect. For example, the ability of anti-CD4 antibodies to block CD4 receptors on T cells makes them effective anti-inflammatories, yet their ability to recruit FcγRs also directs immune attack against the target cells, resulting in T cell depletion. Effector function can also be a problem for radiolabeled antibodies, referred to as radioconjugates, and antibodies conjugated to toxins, referred to as immunotoxins. These drugs can be used to destroy cancer cells, but the recruitment of immune cells via Fc interaction with FcγRs brings healthy immune cells in proximity to the deadly payload (radiation or toxin), resulting in depletion of normal lymphoid tissue along with targeted cancer cells. It has been shown by the applicant that variant according to the invention, while exhibiting altered binding to the C1q and to at least an Fcgamma receptor, display similar other functional characteristic such as antigen recognition or FcRn affinity. Therefore other important antibody properties, such as antibody stability, structural integrity, or ability to interact with other important Fc ligands such as FcRn and proteins A and G, are not perturbed.

The target molecule may be of any kinds and includes both exogenous and endogenous molecules. Target molecules (also called antigens when the polypeptide variant is an antibody) include without being limited, viral, bacterial and fungal proteins, prions, toxins, enzymes, membrane receptors, drugs and soluble proteins.

Membrane receptors include, without being limited to,RhD antigen, CD3, CD4, CD19, CD20, CD22, CD25, CD28, CD32B, CD33, CD38, CD40, CD44, CD52, CD71 (transferrin receptor), CD80, CD86, CTLA-4, CD147, CD160, CD224, growth factor receptors like those belonging to the ErbB family of receptors ErbB1, ErbB2, ErbB3, ErbB4 (EGFR, HER2/neu, HER3, HER4), VEGF-R1, VEGF-R2, IGF-R1, PIGF-R, MHC class I and MHC class II molecules, e.g. HLA-DR, type I interferon receptor, interleukin receptors like IL-1R, IL-2R alpha, IL-2R beta and IL-2R gamma, IL-6R, hormone receptors like Müllerian inhibitory substance type II receptor, LDL receptor, NKp44L, chemokine receptors like CXCR4, CCR5, TNFR, CD137, integrins, adhesion molecules like CD2, ICAM, EpCAM, , G-protein-coupled receptor,

The membrane proteins also include tumour markers like GD2, GD3, CA125, MUC-1, MUC-16, carcinoembrionic antigen (CEA), Tn, glycoprotein 72, PSMA, HMW-MAA other proteins such as BDCA-2 specific for DC cells, glucagon-like peptides (e.g., GLP-1, etc.), enzymes (e.g., glucocerebrosidase, iduronate-2-sulfatase, alpha-galactosidase-A, agalsidase alpha and beta, alpha-L-iduronidase, butyrylcholinesterase, chitinase, glutamate decarboxylase, imiglucerase, lipase, uricase, platelet-activating factor acetylhydrolase, neutral endopeptidase, myeloperoxidase, etc.), interleukin and cytokine binding proteins (e.g., IL-18 bp, TNF-binding protein, etc.), macrophage activating factor, macrophage peptide, B cell factor, T cell factor, protein A, allergy inhibitor, cell necrosis glycoproteins, immunotoxin, lymphotoxin, tumor necrosis factor, tumor suppressors.

Soluble proteins include, without being limited to, cytokines such as for instance IL-1 beta, IL-2, IL-6, IL-12, IL-23, TGF beta, TNF alpha, IFN gamma, chemokines, growth factors like VEGF, G-CSF, GM-CSF, EGF, PIGF, PDGF, IGF, hormones and inhibitory antibody such as a FVIII inhibitory, metastasis growth factor, alpha-1 antitrypsin, albumin, alpha-lactalbumin, apolipoprotein-E, erythropoietin, highly glycosylated erythropoietin, angiopoietins, hemoglobin, thrombin, anti-thrombin III, thrombin receptor activating peptide, thrombomodulin, factor VII, factor VIIa, factor VIII, factor IX, factor XIII, plasminogen activating factor, fibrin-binding peptide, urokinase, streptokinase, hirudin, protein C, C-reactive protein, B cell activating factor receptor, receptor antagonists (e.g., IL1-Ra), complement proteins, C1, C2, C3, C4, C5, C6, C7, C8, C9, factor H, factor I, factor P, other proteins such as CSAP, CD137-ligand, lectins, sialylated proteins.

Variants according to the invention may be used for microbial toxin neutralisation (such as tetanus toxin or anthrax) or to prevent viral infection such as hepatitis, infections mediated by papillomavirus or respiratory syncytial virus. Neutralising variants according to the invention may also be used in case of poisoning. Lastly, neutralising variants according to the invention may also be directed against auto-antigens such as VEGF for the treatment of cancer or other pathologies such as macular degenerescence due to age.

The variants according to the invention may also be used to antagonize membrane receptor such as cytokine receptors, growth factor receptors, integrins. For example said variants may be used as immunosuppressor in transplantation such as an anti-IL-2R (CD25) that may inhibit lymphocyte T proliferation. Said variants may also be used to limit inflammatory processes (inflammatory bowel disease, atherosclerosis, rheumatoid arthritis), such as antibody directed against the αchain of the IL-6 receptor in case of rheumatoid arthritis. Variants of the invention may also be used to inhibit tumoral growth, such as anti-EGFR antibodies or anti-HER-2 receptor antibodies. Anti-integrins variants according to the invention may also be used for limiting thrombosis formation, such as acute coronary syndrome, or for treating psoriasis. In other embodiments, variants according to the invention may also be used for treating multiple sclerosis.

Accordingly variants of the invention may be used as a neutralizing, antagonist or agonist of a target molecule for treating or preventing cancer, inflammatory disorders, infectious disease, auto-immune disease or poisoning.

By "autoimmune diseases" herein include allogenic islet graft rejection, alopecia greata, ankylosing spondylitis, antiphospholipid syndrome, autoimmune Addison's disease, antineutrophil cytoplasmic autoantibodies (ANCA), autoimmune diseases of the adrenal gland, autoimmune hemolytic anemia, autoimmune hepatitis, autoimmune myocarditis, autoimmune neutropenia, autoimmune oophoritis and orchitis, autoimmune thrombocytopenia, autoimmune urticaria, Behcet's disease, bullous pemphigoid, cardiomyopathy, Castleman's syndrome, celiac spruce-dermatitis, chronic fatigue immune disfunction syndrome, chronic inflammatory demyelinating polyneuropathy, Churg-Strauss syndrome, cicatrical pemphigoid, CREST syndrome, cold agglutinin disease, Crohn's disease, dermatomyositis, discoid lupus, essential mixed cryoglobulinemia, factor VIII deficiency, fibromyalgia-fibromyositis, glomerulonephritis, Grave's disease, Guillain-Barre, Goodpasture's syndrome, graft-versus-host disease (GVHD), Hashimoto's thyroiditis, hemophilia A, idiopathic pulmonary fibrosis, idiopathic thrombocytopenia purpura (ITP), IgA neuropathy, IgM polyneuropathies, immune mediated thrombocytopenia, juvenile arthritis, Kawasaki's disease, lichen plantus, lupus erthematosis, Meniere's disease, mixed connective tissue disease, multiple sclerosis, type 1 diabetes mellitus, myasthenia gravis, pemphigus vulgaris, pernicious anemia, polyarteritis nodosa, polychrondritis, polyglandular syndromes, polymyalgia rheumatica, polymyositis and dermatomyositis, primary agammaglobinulinemia, primary biliary cirrhosis, psoriasis, psoriatic arthritis, Reynauld's phenomenon, Reiter's syndrome, rheumatoid arthritis, sarcoidosis, scleroderma, Sjorgen's syndrome, solid organ transplant rejection, stiff-man syndrome, systemic lupus erythematosus, takayasu arteritis, temporal arteristis/giant cell arteritis, thrombotic thrombocytopenia purpura, ulcerative colitis, uveitis, vasculitides such as dermatitis herpetiformis vasculitis, vitiligo, and Wegner's granulomatosis.

By "inflammatory disorders" herein include acute respiratory distress syndrome (ARDS), acute septic arthritis, adjuvant arthritis, allergic encephalomyelitis, allergic rhinitis, allergic vasculitis, allergy, asthma, atherosclerosis, chronic inflammation due to chronic bacterial or viral infections, chronic obstructive pulmonary disease (COPD), coronary artery disease, encephalitis, inflammatory bowel disease, inflammatory osteolysis, inflammation associated with acute and delayed hypersensitivity reactions, inflammation associated with tumors, peripheral nerve injury or demyelinating diseases, inflammation associated with tissue trauma such as burns and ischemia, inflammation due to meningitis, multiple organ injury syndrome, pulmonary fibrosis, sepsis and septic shock, Stevens-Johnson syndrome, undifferentiated arthropy, and undifferentiated spondyloarthropathy.

By "infectious diseases" herein include diseases caused by pathogens such as viruses, bacteria, fungi, protozoa, and parasites. Infectious diseases may be caused by viruses including adenovirus, cytomegalovirus, dengue, Epstein-Barr, hanta, hepatitis A, hepatitis B, hepatitis C, herpes simplex type I, herpes simplex type II, human immunodeficiency virus, (HIV), human papilloma virus (HPV), influenza, measles, mumps, papova virus, polio, respiratory syncytial virus, rinderpest, rhinovirus, rotavirus, rubella, SARS virus, smallpox, viral meningitis, and the like. Infections diseases may also be caused by bacteria including Bacillus antracis, Borrelia burgdorferi, Campylobacter jejuni, Chlamydia trachomatis, Clostridium botulinum, Clostridium tetani, Diptheria, E. coli, Legionella, Helicobacter pylori, Mycobacterium rickettsia, Mycoplasma nesisseria, Pertussis, Pseudomonas aeruginosa, S. pneumonia, Streptococcus, Staphylococcus, Vibria cholerae, Yersinia pestis, and the like. Infectious diseases may also be caused by fungi such as Aspergillus fumigatus, Blastomyces dermatitidis, Candida albicans, Coccidioides immitis, Cryptococcus neoformans, Histoplasma capsulatum, Penicillium marneffei, and the like. Infectious diseases may also be caused by protozoa and parasites such as chlamydia, kokzidioa, leishmania, malaria, rickettsia, trypanosoma, and the like.

Furthermore, variants of the present invention may be used to prevent or treat additional conditions including but not limited to heart conditions such as congestive heart failure (CHF), myocarditis and other conditions of the myocardium; skin conditions such as rosecea, acne, and eczema; bone and tooth conditions such as bone loss, osteoporosis, Paget's disease, Langerhans' cell histiocytosis, periodontal disease, disuse osteopenia, osteomalacia, monostotic fibrous dysplasia, polyostotic fibrous dysplasia, bone metastasis, bone pain management, humoral malignant hypercalcemia, periodontal reconstruction, spinal cord injury, and bone fractures; metabolic conditions such as Gaucher's disease; endocrine conditions such as Cushing's syndrome; and neurological conditions.

In one embodiment of the invention, anti-RhD variants according to the invention may be used for the treatment or the prevention of rhesus incompatibility.

In other embodiments, anti-CD20 variants according to the invention may be used for the treatment of lymphoid leukaemia.

In other embodiments, variants according to the invention may be used for the treatment and/or the prevention of autoimmune diseases such as immune thrombocytopenic purpura, thrombotic thrombocytopenic purpura, rheumatoid polyarthritis and lupus erythematous.

In some embodiments, the parent polypeptide maybe selected from commercial antibodies such as anti-RhD antibodies (see EMAB2® described in FR 09 51412 or MonoRho® of ZLB, Zurich) or anti-CD20 (see WO2006064121)

The parent polypeptide may also be Avastin® (anti-VEGF), Remicade® (anti-TNF-α), Erbitux® (anti-EGFR), Vectibix® (anti-EGFR), Tysabri® (anti-alpha4 chain of integrin), Herceptin® (anti-HER2/neu), the list not being limitative.

In some embodiments the variant is a neutralizing antibody directed to a target molecule selected from the group of membrane receptors, human soluble proteins, toxins, viral, bacterial and fungal proteins.

Because of its low binding to C1q and some FcγRs, the variant of the invention is particularly appropriate to be used for the treatment of conditions in which the recruitment of the immune system through ADCC or CDC is not crucial for the therapeutic efficiency.

The administration of the polypeptide variant of the invention is anticipated to induce less side-effect and less IgG-mediated cytotoxicity than most of the antibodies and immunoadhesins which do not comprise 294Del or 293Del in their Fc region.

A further object of the invention is thus the use of the variant of the invention for preventing or treating a pathological condition wherein the induction of ADCC and/or CDC responses is not desirable.

Therefore certain embodiments of the invention relate to a variant of a parent polypeptide for use in preventing or treating a pathological condition wherein the induction of ADCC and/or CDC response is not desirable, said variant comprising an Fc region, exhibiting reduced binding to the protein C1q and to at least one receptor FcγR as compared to the said parent polypeptide and comprising an amino acid modification selected from 294Del, 293Del and 293Del/294Del within its Fc region, as compared to the Fc region of its polypeptide parent, with the proviso that said variant of the parent polypeptide does not comprise an amino acid modification consisting in 294del or 293del combined to mutations in both positions 307 and 434, the numbering of amino acids in the Fc region referring to the numbering according to the EU index, or equivalent in Kabat.

Other embodiments relates to a a variant of a parent polypeptide for use in preventing or treating a pathological condition wherein the induction of ADCC and/or CDC response is not desirable, said variant comprising an Fc region, exhibiting reduced binding to the protein C1q and to at least one receptor FcγR as compared to the said parent polypeptide and comprising an amino acid modification selected from 294Del, 293Del and 293Del/294Del within its Fc region, as compared to the Fc region of its polypeptide parent, with the proviso that said variant of the parent polypeptide does not consist in 294Del/T307P/N434Y or 293Del/T307P/N434Y variants.

The induction of ADCC and CDC responses is not desirable when the therapeutic efficacy of the variant does not require effector-cell activation or CDC activation. Such a variant includes for example blocking or neutralizing antibodies.

Pathological conditions which treatment or prevention do not require the induction of CDC and ADCC, include without being limited to, graft rejection, autoimmune diseases, inflammatory disorders, infectious diseases or cancer. Another object of the invention is the use of a variant of the invention for preparing a pharmaceutical composition.

In certain embodiments of the invention variants to be used as described above are variants exhibiting reduced binding to at least one protein selected from the protein C1q and receptor FcγRs as compared to the said parent polypeptide and comprising an amino acid modification selected from 294Del, 293Del and 293Del/294Del within its Fc region, as compared to the Fc region of its polypeptide parent.

In particular embodiments of the invention the variant does not consist in the 294Del/T307P/N434Y or 293Del/T307P/N434Y variants.

In some embodiments, the variant of the invention further comprises at least an amino acid modification selected from 378V, 434Y, 397M, 302A, 434S, 315D, 230S, 307A, 228R, 230S/315D/428L/434Y, 2591/315D/434Y and the amino acid modification 256N/378V/383N/434Y in the Fc region.

In some specific embodiments, the Fc region of the variant of the invention is a variant of a wild-type IgG Fc region comprising at least one mutation selected from the group consisting of 294Del, 293Del, 293Del/294Del, 294Del/256N/378V/383N/434Y, 294Del/2591/315D/434Y, 294Del/397M, 294Del/302A, 294Del/434S, 294Del/315D, 294Del/230S, 294Del/307A, 294Del/228R, 230S/315D/428L/434Y, 294Del/378V and 294Del/434Y of Fc region wherein the numbering of the amino acids in the Fc region is that of the EU index, or equivalent in Kabat.

In some other embodiments, the Fc region of the variant is selected from the group of variants of IgG wild-type Fc regions consisting of 294Del, 293Del, 293Del/294Del, 378V/294Del, 434Y/294Del, 294Del/397M, 294Del/302A, 294Del/434S, 294Del/315D, 294Del/230S, 294Del/307A, 294Del/228R, 230S/315D/428L/434Y, 2591/315D/434Y/294Del and 256N/378V/383N/434Y/294Del, wherein the numbering of the amino acids refers to the Fc amino acid numbering of the EU index, or equivalent in Kabat.

A further object of the invention is to provide pharmaceutical compositions comprising the said variant. When the said variant is an antibody, the variant may be present in the form of monoclonal or polyclonal antibodies. The pharmaceutical compositions are prepared by mixing the polypeptide variant having the desired degree of purity with optional physiologically acceptable carrier, excipients or stabilizers in the form of lyophilised formulations or aqueous solutions.

The pharmaceutical composition of the invention may be formulated according to standard methods such as those described in Remington: The Science and Practice of Pharmacy (Lippincott Williams & Wilkins; Twenty first Edition, 2005).

Pharmaceutically acceptable excipients that may be used are, in particular, described in the Handbook of Pharmaceuticals Excipients, American Pharmaceutical Association (Pharmaceutical Press; 6th revised edition, 2009).

In order to treat a patient in need, a therapeutically effective dose of the variant may be administered. By "therapeutically effective dose" herein is meant a dose that produces the effects for which it is administered. The exact dose will depend on the purpose of the treatment, and will be ascertainable by one skilled in the art using known techniques. Dosages may range from 0.001 to 100 mg/kg of body weight or greater, for example 0.1, 1.0, 10, or 50 mg/kg of body weight, with 1 to 10mg/kg being preferred. As is known in the art, adjustments for protein degradation, systemic versus localized delivery, and rate of new protease synthesis, as well as the age, body weight, general health, sex, diet, time of administration, drug interaction and the severity of the condition may be necessary, and will be ascertainable with routine experimentation by those skilled in the art.

Administration of the pharmaceutical composition comprising a variant may be done in a variety of ways, including, but not limited to, orally, subcutaneously, intravenously, parenterally, intranasally, intraortically, intraocularly, rectally, vaginally, transdermally, topically (e.g., gels), intraperitoneally, intramuscularly, intrapulmonary.

The variants described herein may be administered with other therapeutics concomitantly, i.e., the therapeutics described herein may be co-administered with other therapies or therapeutics, including for example, small molecules, other biologicals, radiation therapy, surgery, etc.

The present invention is further illustrated by, without on any way being limited to, the examples below.

### EXAMPLE: Production of IgG variants based on Fc variants and characterization of said IgG.

### I IgG variant production in 293-F mammalian cells

### I.1 Vector construction

Human Fc gene encoding amino acid residues 226-447 (EU index, or equivalent in Kabat) i.e. Fc fragment (Fc226, SEQ ID n°1), derived from a human IgG1 heavy chain (Poul MA et al., Eur. J. Immunol. 25(7): 2005-2009 1995), was cloned into the eukaryotic expression vector pMGM05-R603 (Figure 2) as a *BamHI*/*Notl* fragment using standard PCR protocols. The pMGM05-R603 vector is derived from pCEP4 (Invitrogen) and contains the heavy chain of the R603 chimeric anti-CD20 antibody (LFB-R603). The light chain of this antibody was inserted into a similar vector derived from pCEP4 (pMGM01-R603). All the mutations in the Fc fragment were inserted into the pMGM05-R603 vector by overlap PCR. For instance, the variant 294Del was obtained using two sets of primers. To perform the first PCR, the 5' primer MG_619 5'-*AGTACTGACTCTACCTA*GGATCCTGCCCACCGTGC-3' (SEQ ID N°10) and the 3' primer MG_934 5'-GCTGTTGTACTGCTCCCGCGGCTT-3' (SEQ ID N°11) were used, and for the second PCR, the 5' primer MG_933 5'-AAGCCGCGGGAGCAGTACAACAGC-3' (SEQ ID N°12) and the 3' primer MG_621 5'-*ACTGCTCGATGTCCGTACTA*TGCGGCCGCGAATTC-3' (SEQ ID N°13) were used (where *BamHI* and *Notl* restriction sites are underlined and italic characters correspond to the non-specific tails removed during the cloning step). A fraction of each PCR fragment was then associated and the resulting elongated fragment was amplified by PCR using standard protocols with the primers MG_619 and MG_621. The PCR product was purified on 1% (w/v) agarose gels, digested with *BamHI* and *Notl* restriction enzymes and cloned into the pMGM05-R603 vector. Alternatively, the mutagenesis could be performed with the same primers (MG_933 and MG_934) using the Quick-change Multi kit (Stratagene, La Jolla, CA).

### I. 2 Cell culture production

FreeStyle™ 293-F cells (Invitrogen) were co-transfected with pMGM01-R603 and pMGM05-R603 vectors in equimolar amounts (250ng/ml) with FreeStyle™ MAX reagent (1µl/ml) using standard protocols (Invitrogen). Cells were cultured in suspension in serum-free medium for 7 days post-transfection and IgG containing supernatants were harvested after centrifugation of the cells at 100g for 10 minutes. Supernatants (1ml) were then titrated (1.3) and frozen at -20°C before binding characterization by ELISA (111.1.1).

### I.3 Titration of the IgG variants produced

IgG variants secreted in the supernatants previously harvested were quantified using an ELISA assay on recombinant protein L (Pierce), with purified R603 antibody used as standard. Supernatants and standard antibody, serially diluted in PBS/0.05% Tween-20, were tested on Maxisorp immunoplates (Nunc) previously coated with 0.25µg protein L/well and blocked with 5% skimmed milk in PBS. After incubation for 1 hour at 37°C, wells were washed 3 times with PBS/0.05% Tween-20. Bound IgG variants were detected with an HRP goat anti-human IgG (γ chain specific) F(ab')2 fragment (Sigma). IgG variants produced were quantified (1-4µg/ml) using the standard curve obtained with the purified R603 antibody.

### II IgG variant production in Y2B/O cells

### II.1 Vector construction

The Fc variants 2591/315D/434Y 2591/315D/434Y_294DeI, 256N/378V/383N/434Y and 256N/378V/383N/434Y_DeI294 are prepared in an IgG format with anti-CD20 (R603) or anti- RhD⁺ (R593) specificity in YB2/0 cell line. In order to maximize productivity in the YB2/0 cell line, the full length heavy and light chains cDNA as well as the Fc fragment coding the 2591/315D/434Y and 256N/378V/383N/434Y variants were neosynthesized with codon optimisation for Rattus norvegicus. Unwanted features such as cryptic splicing sites or restriction sites were removed. Only a restriction site (Apal) was present at the junction variable/constant region.

Where needed, 294Del mutation was introduced by assembly PCR by using in PCR1 DELI294P1 (5' primer 5'-CAACGCCAAGACCAAGCCCCGGGAGCAGTACAACAGCACCTACAGGG-3',SEQ ID NO 14) + HCH20GA-Ascl (3' primer 5'- AGCGGCGCGCCTCATCA-3', SEQ ID NO 15) leading to an amplicon of 501 bp, in PCR2 DEL294 P2 (5' primer 5' ACCAAGGGCCCAAGCGTGT -3', SEQ ID NO 17) + HCH20GA-Apal (3' primer 5'-CCCTGTAGGTGCTGTTGTACTGCTCCCGGGGCTTGGTCTTGGCGTTG -3', SEQ ID NO 16) leading to an amplicon of 541 bp, and finally in PCR3 HCH20GA-Ascl and HCH20GA-Apal leading to a PCR product of 998bp. The PCR product was digested with AscI and Apal and the purified 979bp fragment was inserted in the expression construct in replacement of the parental DNA sequence.

### II.2 Cell culture production

Transfections were realized in YB2/0 stable pools. Cells from the YB2/O cell line were electroporated with each linearized expression vector, then diluted at 25,000 cells/mL in EMS medium + 5 % v/v dyalised FCS (InvitroGen) and dispensed under 1 ml/well in 24-well plates. After 3 days of cell recovery, selection pressure was applied by adding G418 at 2g/l final and phenol red 1 % final in EMS + 5 % FCS medium. After 10 days of incubation, 3 pools of 8 P24 wells were made and cells were split at 2.10⁵ cv/ml in F25. Antibody productions were conducted in roller bottles in EMS + 5% FCS with 0.5g/l G418 at a starting concentration between 2 - 8 10⁵ cv/ml and a maximum cell production is achieved on 5 days, supernatant was then collected and titrated in FastElysa (RD Biotech).
Antibodies were purified on protein A Sepahrose type HiTrap protein A FF (GE-Helthcare) then eluted in sodium citrate buffer 25 mM, pH 3.0 and fractions were neutralized, dialyzed into PBS, pH 6.0 overnight at 4 °C.
The purified IgGs were characterised by SDS-PAGE under non-reducing and reducing conditions as well as with gel filtration in order to estimate aggregate contents. Whatever the mutations, IgGs were purified to greater than 85 % and most often to greater than 95 % and displayed the characteristic heavu and light chain bands for each IgG. LAL endotoxin test (Limulus Amebocyte Lysate) Gel Clot method was further used to test purified IgGs for the presence of endotoxins. The law endotoxin levels as well as the low aggregate content demonstrate that the produced antibodies are therefore compatible for functional testing.

### III. Binding characterization of the IgG variants by ELISA

### III.1 Materials and methods

### III.1.1 ELISA tests of IgG variants produced in the supernatants of 293-F cells

The IgG variants were tested for their binding to several receptors by ELISA: C1q complement (Calbiochem), FcγRIIIaV158 (R&D system), FcRn-p3 (FcRn-p3 refers to a fusion protein comprising the β2 microglobulin chain and the α-chain fused to the bacteriphage protein p3 and the CVDE protein and is produced in a baculovirus system as described in Popov et al., Mol. Immunol.,1996, 33:521-530) FcγRIIaR131 (R&D system), FcγRI (R&D system) and FcγRIIb (R&D system). ELISA tests were performed in PBS for all receptors except for FcRn which was realised in P6 (sodium phosphate 100mM, sodium chloride 50mM pH6.0) as FcRn/IgG binding is pH-dependent and optimum at pH6.0. Maxisorp immunoplates were coated with 0.5µg C1q complement/well in PBS, 0.25µg FcγRIIIaV158/well in PBS or 0.1µg FcγRI/well in PBS or 0.1µg FcRn-p3/well in P6. Immobilizer nickel chelate plates (Nunc) were coated with 0.1µg FcγRIIaR131/well or 0.4µg FcγRIIb/well in KCI 0.01M. After coating overnight at 4°C, plates were washed 2 times with PBS/0.05% Tween-20 (or P6/0.05% Tween-20 for FcRn) and saturated with PBS/4% BSA (or P6/4%BSA for FcRn) for 2 hours at 37°C. In parallel, supernatants were diluted in PBS at a final IgG concentration of 0.5µg/ml (or diluted in P6 at 03µg/ml for FcRn binding test) and mixed with HRP F(ab')2 goat anti-human F(ab')2 at the same concentration for 2 hours at room temperature. F(ab')2-aggregated IgGs were then incubated under gentle agitation for 1 hour on the saturated ELISA plates without dilution for C1q, FcγRIIaR131 and FcγRIIb (i.e IgGs at 0.5µg/ml), diluted in PBS at 0.25µg/ml for FcγRIIIaV158 and FcγRI, or diluted in P6 at 0.0375µg/ml for FcRn-p3. Plates were then revealed with TMB (Pierce) and absorbance read at 450nm.

### 111.1.2 ELISA tests of purified IgG variants

The IgG variants produced in Y2B/0 were tested for their binding to several receptors by ELISA: FcRn-p3 (as in 11.1.1), FcγRI (R&D system), FcγRIIIaV158 (R&D system), FcγRIIIaF158 (produced transiently by PX Therapeutics in HEK293F cells as His-tagged protein), FcγRIIaR131 (R&D system), FcγRIIaH131 (produced transiently by PX Therapeutics in HEK293F cells as His-tagged protein) and FcγRIIb (R&D system). ELISA tests were performed in PBS for all receptors except for FcRn which was realised in P6 (sodium phosphate 100mM, sodium chloride 50mM, pH6.0). Maxisorp immunoplates (Nunc) were coated with 100ng recombinant protein/well in PBS for FcγRI, FcγRIIIaV158, FcγRIIIaF158 and FcγRIIaH131 or 200ng FcRn-p3/well in P6. Immobilizer nickel chelate plates (Nunc) were coated with 100ng FcγRIIaR131/well or 400ng FcγRIIb/well in KCI 0.01 M. After coating overnight at 4°C, plates were washed 2 times with PBS/0.05% Tween-20 (or P6/0.05% Tween-20 for FcRn) and saturated with PBS/4% BSA (or PBS/4% skimmed milk for FcγRI or P6/4% skimmed milk for FcRn) for 2 hours at 37°C.

For FcγRIIIaV158, FcγRIIIaF158, FcγRIIaR131, FcγRIIaH131 and FcγRIIb binding tests, purified IgGs were diluted in PBS at a final concentration of 2-4µg/ml and mixed with HRP F(ab')2 goat anti-human F(ab')2 at the same concentration for 2 hours at room temperature. F(ab')2-aggregated IgGs were then incubated under gentle agitation for 1 hour at 30°C on the saturated ELISA plates after serial dilution in PBS. Plates were then revealed with TMB (Pierce) and absorbance read at 450nm. For FcγRI and FcRn, a direct ELISA was performed. Purified IgGs were diluted in PBS (or P6 for FcRn) supplemented with 4% skimmed milk and 0.01%Tween 20 and incubated on plates for 2 hours. Bound antibodies were then detected with HRP F(ab')2 goat anti-human F(ab')2 (1/2500) diluted in the same buffer. After 2 hours incubation at 37°C, plates were revealed with TMB (Pierce) and absorbance read at 450nm.

### III.2 Results

### III.2.1 IgG produced in supernatants of 293-F cells

For each IgG variant, two independent experiments were carried out (production, supernatant titration and ELISA on the 5 receptors). ELISA results were expressed as a ratio of specific signal (OD450nm) obtained for the IgG variant compared to the signal of the IgG-WT. These ELISA tests showed that the mutation 294Del drastically impairs the capacity of the variants to bind C1q (ratio/IgG-WT<0.50) and the FcγRs (ratio/IgG-WT<0.25) as compared to their respective parent polypeptides. On the contrary, the said mutation only results in around 0-35% loss of FcRn binding for the variants combining the 294Del with at least another mutation. The results are presented hereunder in the Tables 1 and 2.

**Table 1: Ratio of the specific signal of IgG1 variants with the specific signal obtained for wild-type IgG1 in ELISA assays for the target proteins C1q, FcγRIIaR131, FcγRIIb, FcγRIIIaV158, FcγRI and FcRn.**

| | **C1q** | | **FcγRI** | | **FcγrRIIaR131** | | **FcγRIIb** | | **FcγRIIIaV158** | | **FcRn** | |
|---|---|---|---|---|---|---|---|---|---|---|---|---|
| **Mutations** | **Ratio/IgG-Wt** | **SD** | **Rations/IgG-WT** | **SD** | **Ratio/IqG-WT** | **SD** | **Ratio/IgG-WT** | **SD** | **Ratio/IgG-WT** | **SD** | **Ratio/IgG-WT** | **SD** |
| **294Del** | 0.10 | 0.10 | 0.37 | 0.01 | 0.01 | 0.00 | 0.01 | 0.00 | 0.01 | 0.01 | 0.44 | 0.02 |
| **259I/315D/434Y** | 1.26 | 0.08 | 0.82 | 0.08 | 1.23 | 0.01 | 1.07 | 0.32 | 1.08 | 0.04 | 4.32 | 1.02 |
| **259I/294Del/315D/434Y** | 0.13 | 0.10 | 0.34 | 0.01 | 0.01 | 0.01 | 0.01 | 0.01 | 0.02 | 0.00 | 2.87 | 0.33 |
| **256N/378V/383N/434Y** | 3.65 | 0.63 | 0.93 | 0.2 | 2.59 | 0.44 | 4.77 | 1.17 | 2.09 | 0.51 | 4.63 | 1.15 |
| **256N/294Del/378V/383N/434Y** | 0.14 | 0.04 | 0.61 | 0.03 | 0.01 | 0.01 | 0.01 | 0.01 | 002 | 0.02 | 3.47 | 0.31 |
| **307A** | 2.24 | 0.51 | 1.20 | 0.11 | 1.8 | 0.01 | 1.47 | 0.49 | 1.14 | 0.19 | 1.99 | 0.45 |
| **2940EL/307**A | 0.46 | 0.00 | 0.35 | 0.02 | 0.01 | 0.00 | 0.20 | 0.00 | 0.01 | 0.00 | 1.94 | 0.54 |
| **228R** | 0.80 | 0.09 | 0.92 | 0.19 | 1.02 | 0.06 | 1.59 | 0.03 | 1.03 | 0.23 | 1.11 | 0.29 |
| **228R/294DEL** | 0.45 | 0.09 | 0.42 | 0.07 | 0.01 | 0.01 | 0.03 | 0.03 | 0.01 | 0.01 | 1.38 | 0.33 |
| **230S** | 0.72 | 0.13 | 0.95 | 0.26 | 0.73 | 0.25 | 0.92 | 0.26 | 0.79 | 0.01 | 1.21 | 0.41 |
| **230S/291DEL** | 0.46 | 0.09 | 0.40 | 0.14 | 0.01 | 0.01 | 0.05 | 0.03 | 0.01 | 0.00 | 1.28 | 0.37 |
| **230S/315D/428L434Y** | 1.12 | 0.29 | 0.91 | 0.16 | 1.45 | 0.34 | 1.17 | 0.07 | 0.40 | 010 | 3.94 | 0.91 |
| **230S/294DEL/315D/428L/434Y** | 0.35 | 0.04 | 0.25 | 0.05 | 0.01 | 0.01 | 0.02 | 0.02 | 0.01 | 0.00 | 3.86 | 0.62 |
| **315D** | 0.95 | 0.28 | 0.71 | 0.24 | 0.77 | 0.34 | 1.02 | 0.56 | 1.25 | 0.15 | 1.09 | 0.21 |
| **294DEL/315D** | 0.29 | 0.14 | 0.40 | 0.09 | 0.01 | 0.00 | 0.21 | 0.07 | 0.01 | 0.01 | 1.16 | 0.19 |
| **378V** | 1.71 | 0.37 | 1.12 | 0.34 | 3.37 | 0.68 | 8,00 | 1.17 | 2.88 | 0.24 | 1.82 | 0.14 |
| **294DEL/378V** | 0.37 | 0.08 | 0.33 | 0.03 | 0.06 | 0.01 | 0.11 | 0.11 | 001 | 000 | 1.46 | 0.09 |
| **434S** | 0.85 | 0.12 | 0.94 | 0.11 | 0.95 | 0.15 | 1.56 | 0.32 | 1.22 | 0.08 | 2.68 | 0.49 |
| **294DEL/434S** | 0.25 | 0.25 | 0.49 | 0.02 | 0.02 | 0.02 | 0.05 | 0.05 | 001 | 000 | 2.64 | 0.60 |
| **302A** | 0.88 | 0.22 | 0.96 | 0.33 | 2.01 | 0.00 | 3.45 | 0.00 | 0.55 | 0.14 | 091 | 0.35 |
| **294DEL/302A** | 0.21 | 0.07 | 0.06 | 0.02 | 0.02 | 0.02 | 0.06 | 0.06 | 0.01 | 0.00 | 0.66 | 0.06 |
| **397M** | 3.70 | 0.79 | 1.69 | 0.34 | 3.77 | 0.19 | 3.12 | 1.28 | 1.87 | 0.16 | 1.01 | 0.14 |
| **294DEL/397M** | 0.25 | 0.25 | 0.68 | 0.23 | 0.03 | 0.02 | 0.04 | 0.04 | 0.01 | 0.01 | 0.84 | 0.13 |
| **434Y** | 1.84 | 0.59 | 0.70 | 0.36 | 1.51 | 0.34 | 1.24 | 022 | 0.80 | 0.26 | 2.53 | 0.70 |
| **294DEL/434Y** | 0.45 | 0.20 | 0.32 | 0.12 | 0.03 | 0.01 | 0.03 | 0.02 | 0.01 | 0.00 | 3.37 | 0.35 |

**Table 2: Impact of 294Del on the binding of IgG1 variants to FcγR, C1q and FcRn receptors: the ratios obtained by dividing the specific signal for the variant obtained by the introduction of 294Del with that of the corresponding IgG1 which does not comprise 294Del are shown in the table hereunder for each protein of interest.**

| **Mutations** | **C1q** | **FcγRI** | **FcγRIIaR131** | **FcγRIIb** | **FcγRIIIaV158** | **FcRn** |
|---|---|---|---|---|---|---|
| **294Del** | 0.10 | 0.37 | 0.01 | 0.01 | 0.01 | 0.44 |
| **259I/294Del/315D/434Y** | 0.10 | 0.41 | 0.01 | 0.01 | 002 | 0.66 |
| **256N/294Del/378V/383N/434Y** | 0.04 | 0.66 | 0004 | 0.002 | 001 | 0.75 |
| **294DEL/307A** | 0.21 | 0.29 | 0.08 | 0.14 | 0.009 | 0.97 |
| **228R/294DEL** | 0.56 | 0.46 | 0.01 | 0.02 | 0.01 | 1.24 |
| **230S/294DEL** | 0.64 | 0.42 | 001 | 0.05 | 0.01 | 1.06 |
| **230S/294DEL/315D/428L/434Y** | 031 | 0.27 | 0.007 | 0.02 | 003 | 0.98 |
| **294DEL/315D** | 031 | 0.56 | 0.01 | 0.21 | 0.008 | 1.06 |
| **294DEL/378V** | 022 | 0.29 | 0.02 | 0.01 | 0.003 | 0.80 |
| **294DEL/434S** | 029 | 0.52 | 0.02 | 0.03 | 0.008 | 0.99 |
| **2294DEL/302A** | 0.24 | 0.06 | 0.01 | 0.02 | 0.02 | 0.73 |
| **294DEL/397M** | 0.07 | 0.40 | 0.008 | 0.01 | 0.005 | 0.83 |
| **294DEL/434Y** | 0.24 | 0.46 | 0.02 | 0.02 | 0.01 | 1.33 |

### III.2.2 ELISA results on purified IgGs

ELISA results were expressed as a ratio of specific signal (OD450nm) obtained for the purified IgG variants obtained from Y2B/0 cells compared to the signal of the IgG-WT (R603, chimeric anti-CD20 antibody and R593, human anti-RhD⁺ antibody) at a single antibody concentration (05µg/ml for FcγRIa and FcγRIIIaV158, 1/µg/ml for FcγRIIIaF158, FcγRIIaR131 and FcγRIIaH131, 2µg/ml for FcγRIIb and 5µg/ml for FcRn). These ELISA tests showed that the mutation 294Del does not significantly modify the binding of variants to FcRn as compared to the corresponding wild type IgG. When compared to variant IgGs comprising the respective parent polypeptide (R603 or R593 2591/315D/434Y and R603 or R593 256N/378V/383N/434Y) the said mutation only results in around 0-30% loss of FcRn binding for the variants 259I/294DeI/315D/434Y and 256N/294Del/378V/383N/434Y (Table 4). On the contrary, the mutation 294Del drastically impairs the capacity of the variants to bind the FcγRs as compared to their respective parent polypeptides.

**Table 3: Ratio of the specific signal of IgG1 variants with the specific signal obtained for wild-type IgG1 in ELISA assays for the target proteins FcγRIa, FcγRIIIaV158, FcγRIIIaF158, FcγRIIaR131, FcγRIIaH131, FcγRIIb and FcRn.**

| Variant | FcgRI | FcgRIIIaV158 | FcgRIIIaF158 | FcgRIIaR131 | FcgRIIaH131 | FegRIIb | FcRn |
|---|---|---|---|---|---|---|---|
| R603-294Del | 0.15 | 0.07 | 0.09 | 0.07 | 0.10 | 0.42 | 0.71 |
| R603-256N/378V/383N/434Y | 2.19 | 1.22 | 1.25 | 1.40 | 0.93 | 1.83 | 8.51 |
| R603-256N/294DeI/378V/383N/434Y | 0.15 | 0.07 | 0.08 | 0.08 | 0.08 | 0.46 | 8.89 |
| R603-259I/315D/434Y | 1.12 | 1.11 | 1.11 | 1.11 | 0.99 | 1.54 | 6.93 |
| R603-259I/294DeI/315D/434Y | 0.10 | 0.05 | 0.08 | 0.07 | 0.08 | 0.41 | 5.97 |
| R593-294Del | 0.08 | 0.07 | 0.06 | 0.10 | 0.10 | 0.18 | 0.91 |
| R593-256N/378V/383N/434Y | 2.19 | 0.98 | 0.92 | 1.08 | 1.01 | 1.55 | 7.49 |
| R593-256N/294Del/378V/383N/434Y | 0.12 | 0.10 | 0.10 | 0.17 | 0.17 | 0.27 | 7.48 |
| R593-259I/315D/434Y | 1.08 | 0.95 | 0.94 | 1.05 | 0.94 | 1.41 | 7.93 |
| R593-259I/294DeI/315D/434Y | 0.13 | 0.07 | 0.07 | 0.12 | 0.10 | 0.18 | 7.19 |

**Table 4: Impact of 294Del on the binding of IgG1 variants to FcγRs and FcRn: the ratios were calculated by dividing the specific signal for the variant obtained by the introduction of 294Del with that of the corresponding IgG1 which does not comprise 294Del.**

| Variant | FcgRI | FcgRIIIaV158 | FcgRIIIaF158 | FcgRIIaR131 | FcgRIIaH131 | FcgRIIb | FcRn |
|---|---|---|---|---|---|---|---|
| R603-294Del | 0.15 | 0.07 | 0.09 | 0.07 | 0.10 | 0.42 | 0.71 |
| R603-256N/294Del/378V/383N/434Y | 0.07 | 0.06 | 0.06 | 0.06 | 0.09 | 0.25 | 1.04 |
| R603-259I/294Del/315D/434Y | 0.09 | 0.05 | 0.07 | 0.06 | 0.08 | 0.27 | 0.86 |
| R593-294Del | 0.08 | 0.07 | 0.06 | 0.10 | 0.10 | 0.18 | 0.91 |
| R593-256N/294Del/378V/383N/434Y | 0.05 | 0.10 | 0.11 | 0.16 | 0.17 | 0.17 | 1.00 |
| R593-259I/294Del/315D/434Y | 0.12 | 0.07 | 0.07 | 0.11 | 0.11 | 0.13 | 0.91 |

### IV Binding characterisation by SPR (Surface Plasmon Resonance) assays

### IV.1 Materials and methods

The interaction of IgG variants (see II.2) with immobilized FcRn was monitored on a BIAcore X100 instrument using a CM5 sensor chip (Biacore, GE Healthcare). The methodology was similar to that previously described for analyzing Fc-FcRn interactions (Popov S. et al., Mol Immunol. 33(6):521-530 (1996)). Recombinant soluble FcRn was coupled to flow cell 2 of the sensor chip using amine-coupling chemistry. The flow cells were activated for 3 min with a 1:1 mixture of 0.1 M N-hydroxysuccinimide and 0.1 M 3-(N,N-dimethylamino)propyl-N-ethylcarbodiimide at a flow rate of 30 µl/min. Recombinant human FcRn (5 µg/ml in 10 mM sodium acetate, pH 5.0) was injected over flow cell 2 for 3 min at 10 µl/min, which resulted in a surface density of 236 response units (RU). Surfaces were blocked with a 3-min injection at 30 µl/min of 1 M ethanolamine-HCl, pH 8.5. Flow cell 1 was used as a control surface without FcRn and was prepared similarly to sample flow cell. The data from this blank flow cell were subtracted from the sample data.
IgG variants were diluted in PBS/Tween-20 (50 mM phosphate buffer, pH 6.0, 150 mM NaCl, 0.02% NaN3, 0.05% Tween-20,) which is used as running buffer in equilibrium binding experiments. All measurements were performed at 25°C with Fc fragment concentrations typically of 0, 8.75, 35, 70 and 200 nM at a flow rate of 10 µl/min.
Data were collected for 8 min and 30s pulse of PBS, pH 7.5 containing 0.05% Tween-20 was used to regenerate the surfaces.
Sensorgrams were generated and analyzed by using BIAevaluation software version 3.1. The equilibrium RU observed for each injection was plotted against the concentration of Fc. The equilibrium KD values were derived by analysis of the plots by using the kinetic affinity model included in the BIA evaluation software.

### IV.2 Results

The binding affinity (KD values) of R593 and R603 antibodies were 190 and 99 nM respectively. In comparison in the context of R603, the KD values of 259V/315D/and in the context of R603 was 26 nM for 259V/315D/434Y illustrating an increased affinity for FcRn between 3 to 6 fold at pH 6.0. The KD values of 259V/294Del/315D/434Y was 28 nM and 20nM for R593 and R603 respectively showing that the 294Del does not significantly modify the affinity to FcRn as compared to the respective parent polypeptide variant.

**Table 5: Impact of Del294 on the binding affinity (KD values) between anti-RhD⁺ (R593) or anti-CD20 (R603) IgG1 variants and FcRn. The ratios were calculated by dividing the KD for with that of the corresponding variant obtained by the introduction of 294Del with that of the corresponding.**

| | **KD (M)** | **Ratio WT/IgG** |
|---|---|---|
| **R603** | **9,90E-08** | **1,00** |
| **R603 294Del** | **1,9E-07** | **0,52** |
| **R603-259I/315D/434Y** | **2,69E-08** | **3,68** |
| **R603-259I/294Del/315D/434Y** | **2,80E-08** | **3,54** |
| **R593** | **1,9E-07** | **1,00** |
| **R593-Del294** | **2,04E-07** | **0,93** |
| **R593-256N/378A/383N/434Y** | **4,20E-08** | **4,52** |
| **R593-256N/294Del/378A/383N/434Y** | **4,23E-09** | **44,92** |
| **R593-259I/315D/434Y** | **2,04E-08** | **9,31** |
| **R593-259I/294Del/315D/434Y** | **2,02E-08** | **9,41** |

### V Functional characterisation

### IV.1 Materials and methods

### IV.1.1 Antigen Binding

2 x 105 cells (RhD+ red blood cells or Raji) were incubated with 100 µl of antibody at different concentrations (0 to 100 µl/ml, final concentration) at 4°C for 30 minutes.

After washing, mAbs are visualized with a goat anti-human Fc gamma coupled to phycoerythrin (100 µl of a dilution of 1:100) at 4°C for 30 minutes. The cells were washed and studied with flow cytometer (FC500, Beckman Coulter).

### IV.1.2 ADCC assay Anti-RhD+

Lymphocytes were prepared from mononuclear cell fraction obtained from 3 individuals buffy-coat by density gradient centrifugation over Ficoll. Pack Plus (Pharmacia). Platelets were removed by centrifugation (190g, 15min) and residual cells were lysed in NH4CI. The monocytes were depleted by two successive adherences (2X30min) to plastic tissue culture flasks at 37°C in IMDM/FCS25%. Final percentage of monocytes should be less than 15% of total cells count. The non adherent lymphocytes were washed before resuspension at 8x107cells /ml in IMDM. Red cells from therapeutic concentrate (group O, Rhesus +) were treated for 10 min with papaïn (1mg/ml) then washed three times with saline solution before resuspension at 4x107cells /ml in IMDM. Human immunoglobulin solution from therapeutic IV Ig (Tégéline, LFB) were diluted at 2 and 10mg/ml in IMDM. The assay was performed in 96 wells microtiter plates (NUNC). Culture supernatants or purified anti-D antibodies (100µl at 200ng/ml in IMDM containing 0.5% FCS), effector cells (25µl), red cells (25µl) and human immunoglobulin (50µl) were incubated for 16h at 37°C in humidified, CO2 %-enriched atmosphere. Non specific release consisting of IMDM in place of the effectors cells suspension was included for each sample tested. After centrifugation of the plates, 60µl of supernatant per well were collected then mixed with 60µl of 2.7diaminofluorenee (DAF, Sigma). After 5 min, OD was measured at 620nm The percent of lysis was estimated using a calibration curve reconstituted with various dilutions of lysed red cells (NH4CI) corresponding to 100, 75, 50, 25 and 0% of lysis respectively.

### IV.1.3 ADCC assay anti-CD20

Lymphocytes were prepared from mononuclear cell fraction obtained from 1 individual buffy-coat by density gradient centrifugation over Ficoll Pack Plus (Pharmacia). Platelets were removed by centrifugation (190g, 15min) and residual red cells were lysed in NH4CI. NK cells were purified using a negative selection isolation kit (Miltenyi Biotec, Bergisch Gladbach, Germany). Purified cells were at least 90% CD56+. Less than 3% cells were stained with PE-conjugated anti-CD14, anti-CD3 or anti-CD19 antibodies. FcγRIIIA polymorphism was determined as previously described (Dall'Ozzo et al, 2003). Raji lymphoblastoid B cells (targets) cultured in RPMI-1640 supplemented with 10% FCS, 2 mmol/l L-glutamine, and 1 mmol/l sodium pyruvate (complete medium). Target cells were mixed with NK cells at an effector-target (E/T) ratio of 15/1 in presence of monoclonal antibody (range, 500-5 ng/ml). The release of LDH in supernatants was measured was measured by fluorimetry (Roche Applied Sciences Cytotoxicity Detection Kit réf 11644793001) using a microplate spectrofluorimeter (Tecan, Mannedorf-Zurich, Switzerland). Data were expressed as arbitrary optical density or fluorescent units (AFU) and percent lysis was calculated according to the following formula:% lysis = 100 x (ER - SR) / (MR - SR), where ER, SR, and MR represent experimental, spontaneous, and maximum release, respectively. ADCC values were expressed as:% ADCC = (% lysis in the presence of mAb) - (% lysis without mAb).

### IV.1.4 CDC assay

Targets ASC-1 cell lines were incubated with increasing concentrations of anti-AMHRII antibodies (0 to 2500 ng/ml) in the presence of baby rabbit serum as a source of complement (dilution to 1/10). After 1 hour of incubation at 37°C, the quantity of LDH released in the supernatant by the lysed target cells was measured by fluorimetry (Roche Applied Sciences Cytotoxicity Detection Kit ref. 11644793001) and used to calculate the percentage of complement-dependent cytotoxicity mediated by the different antibodies. The percent lysis was calculated according to the following formula : % lysis = ER - SA, where ER is the experimental response and SA the spontaneous activity obtained when target cell is incubated in presence of complement, without antibody. Results are expressed as the percent of lysis as a function of the antibody concentration. Emax (percentage of maximum lysis) and EC50 (quantity of antibody that induces 50% of maximum lysis) were calculated using PRISM software.

### IV.2 Results

Antigen recognition test show that introduction of Del294 mutation either in wt or in variant parent anti-RhD⁺IgG1 does not affect the antigen binding properties of the IgG1 variants obtained. Indeed all anti-RhD⁺ (R593) IgGs variants bind to RhD⁺ RBCs (red blood cells) as illustrated in table 6 below.

**Table 6: Binding of anti-RhD⁺ IgG1 wt (R593) and variants to RBCs expressing the RhD⁺ antigen.**

| | **Ag binding** |
|---|---|
| **Anti-RhD⁺ IgG1 variants** | **MFI (Arbitrary Unit)** |
| T256N/A378V/S383N/N434Y | 49,6 |
| V259I/N315D/N434Y | 47,2 |
| E294Del | 52,6 |
| E294Del/T256N/A378V/S383N/N434Y | 44,7 |
| E294Del/V259I/N315D/N434Y | 47,9 |
| R593 | 31,7 |

Furthermore, consistent with the very low affinity of 259I/294Del/315D/434Y and 256N/294Del/378V/383N/434Y and 294Del variants for Fcy receptors, said variants displays no ADCC activity as compared to R593 or parent variant IgGs as illustrated in table 7

**Table 7: Anti-RhD⁺ 294Del variants display no ADCC activity. Half maximal effective concentration inducing 50 % lysis of target cells (EC50, ng/ml) and percentage of maximal target cell lysis in the presence of anti-CD20 variants and wt, as estimated in IV.1.2.**

| | **ADCC** | |
|---|---|---|
| **Anti-RhD+ IgG1 variants** | **EC50 (ng/ml)** | **Max. Lysis (%)** |
| T256N/A378V/S383N/N434Y | 11,06 | 93 |
| V259I/N315D/N434Y | 14,17 | 89 |
| E294Del | 75,86 | 5 |
| E294Del/T256N/A378V/S383N/N434Y | 75,86 | 6 |
| E294Del/V259I/N315D/N434Y | 75,86 | 4 |
| R593 | 11,06 | 93 |

It has further been shown (see table 8 below) that introduction of Del294 mutation does not affect the binding of a wt anti-CD20 (R603) IgG1 or a V259I/N315D/N434Y anti-CD20 (R603) IgG1 variant for the CD20 antigen.

**Table 8: Binding of anti-CD20 IgG1 wt (R603) and R603_259I/315D/434Y, R603_259I/294Del/315D/434Y and R603_294Del IgG variants to Raji lymphoblastoid B cells expressing the CD20 antigen.**

| | **Ag binding** |
|---|---|
| **Anti-CD20 IgG1 variants** | **MFI (Arbitrary Unit)** |
| Fcwt | 123 |
| V259I/N315D/N434Y | 137 |
| E294Del | 126 |
| E294Del/V259I/N315D/N434Y | 96 |

As expected in view of the very low affinity of R603_259I/294Del/315D/434Y and R603_294Del IgG variant for both the C1q protein and the Fcγ receptors, the said variants displays nor ADCC nor CDC activity as compared to R603 or parent variant IgGs as illustrated in table 9 and 10 below.

**Table 9: Anti-CD20 294Del variants display no ADCC activity. Half maximal effective concentration inducing 50 % lysis of target cells (EC50, ng/ml) and percentage of maximal target cell lysis in the presence of anti-CD20 variants and wt, as estimated in IV.1.3.**

| | **CDC** | |
|---|---|---|
| **Anti-CD20 IgG1 variants** | **EC50 (ng/ml)** | **Max. Lysis (%)** |
| Fcwt | 464 | 45 |
| V259I/N315D/N434Y | 410 | 40 |
| E294Del | NA | 2 |
| E294Del/V259I/N315D/N434Y | NA | 1 |

**Table 10: Anti-CD20 294Del variants display no CDC activity. Half maximal effective concentration inducing 50 % lysis of target cells (EC50, ng/ml) in the presence of anti-CD20 variants and wt, as estimated in IV.1.4.**

| | **CDC** |
|---|---|
| **Anti-CD20 IgG1 variants** | **EC50 (ng/ml)** |
| Fcwt | 464 |
| V259I/N315D/N434Y | 410 |
| E294Del | NA |
| E294Del/V259I/N315D/N434Y | NA |

**Table 11: Sequences cited in the sequence listing**

| **SEQ ID NO:** | **Sequences** |
|---|---|
| 1 | Human IgG1 Fc (residues 226-447 according to EU index or equivalent in Kabat) |
| 2 | Human IgG2 Fc |
| 3 | Human IgG3 Fc |
| 4 | Human IgG4 Fc |
| 5 | Fragment of heavy chain of human IgG1 G1m1,17 allotype (shown in Figure 1) |
| 6 | Fragment of heavy chain of human IgG1 G1m3 allotype (shown in Figure 1) |
| 7 | Fragment of the heavy chain of human IgG2 (shown in Figure 1) |
| 8 | Fragment of the heavy chain of human IgG3 (shown in Figure 1) |
| 9 | Fragment of the heavy chain of human IgG4 (shown in Figure 1) |
| 10 | 5'-Primer |
| 11 | 3'-Primer |
| 12 | 5'-Primer |
| 13 | 3'-Primer |
| 14 | 5'-Primer |
| 15 | 3'-Primer |
| 16 | 3'-Primer |
| 17 | 5'-Primer |

## Claims

1. A method for producing a variant of a parent polypeptide comprising a Fc region of SEQ ID NO:1, which variant exhibits reduced binding to the protein C1q and to at least one receptor FcgR as compared to the said parent polypeptide, wherein an amino acid modification selected from the group consisting of:
(i) 294Del, wherein said amino acid modification 294del is the sole amino acid modification introduced in the Fc region of the parent polypeptide,
(ii) 293Del, and
(iii) 293Del/294Del,
is introduced within the Fc region of the parent polypeptide, the numbering of amino acids in the Fc region referring to the numbering according to the EU index, or equivalent in Kabat.

2. The method according to claim 1, wherein the amino acid modification 293Del is the sole amino acid modification introduced in the Fc region of the parent polypeptide.

3. The method according to claim 1, wherein the amino acid modification del293/del294 is the sole amino acid modification introduced in the Fc region of the parent polypeptide.

4. The method according to anyone of claims 1 to 3 wherein the Fc region of the parent polypeptide is a variant of a wild-type IgG Fc region comprising an amino acid modification selected from 434Y, 378V, 259I/315D/434Y and 256N/378V/383N/434Y.

5. The method according to anyone of claims 1 to 4 wherein the variant is selected from the group consisting of isolated Fcs, Fc-fusion proteins, Fc-conjugates and antibodies.

6. The method according to anyone of claims 1 to 5 wherein the step of introducing the said amino acid modification within the Fc region of the parent polypeptide comprises:
(i) providing a nucleic acid encoding the parent polypeptide,
(ii) modifying the nucleic acid provided in step (i) so as to obtain a nucleic acid encoding for the said variant, and
(iii) expressing the nucleic acid obtained in step (ii) in a host cell and recovering the said variant.

7. A variant of a parent polypeptide comprising a Fc region of SEQ ID NO:1, and exhibiting reduced binding to the protein C1q and to at least one receptor FcgR chosen from FcγRII and FcγRIII as compared to the said parent polypeptide, and wherein an amino acid modification consisting of 293del/294del is introduced within the Fc region of the parent polypeptide, the numbering of amino acids in the Fc region referring to the numbering according to the EU index, or equivalent in Kabat.

8. The variant according to claim 7 wherein the variant is a neutralizing antibody directed to a target molecule selected from the group consisting of membrane receptors, human soluble proteins, toxins, viral, bacterial and fungal proteins.

9. An isolated nucleic acid encoding a variant as defined in anyone of claims 7 to 8.

10. A vector comprising the nucleic acid of claim 9.

11. A host cell containing the vector of claim 10.

12. A variant of a parent polypeptide for use in preventing or treating a pathological condition wherein the induction of antibody-dependent cell-mediated cytotoxicity (ADCC) and/or complement-dependent cytotoxicity (CDC) response is not desirable, said variant comprising an Fc region, exhibiting reduced binding to the protein C1q and to at least one receptor FcgR as compared to the said parent polypeptide and comprising an amino acid modification selected from 294Del, 293Del and 293Del/294Del within its Fc region, as compared to the Fc region of SEQ ID NO:1 of its polypeptide parent, with the proviso that said variant of the parent polypeptide does not consist in 294Del/T307P/N434Y or 293Del/T307P/N434Y variants, the numbering of amino acids in the Fc region referring to the numbering according to the EU index, or equivalent in Kabat.

13. A pharmaceutical composition comprising a variant as defined in anyone of claims 7 or 8.

## Patentansprüche

1. Verfahren zur Herstellung einer Variante eines Vorläuferpolypeptids, das eine Fc-Region der SEQ ID NO: 1 umfasst, wobei diese Variante im Vergleich zum genannten Vorläuferpolypeptid eine reduzierte Bindung an das Protein C1q und an mindestens einen Rezeptor FcgR aufweist, wobei innerhalb von der Fc-Region des Vorläuferpolypeptids eine Aminosäuremodifikation ausgewählt aus der Gruppe bestehend aus:
(i) 2940eI, wobei die Aminosäuremodifikation 294deI die einzige Aminosäuremodifikation ist, die in die Fc-Region des Vorläuferpolypeptids eingeführt ist,
(ii) 293Del, und
(iii) 293Del/294Del,
eingeführt ist, wobei sich die Nummerierung der Aminosäuren in der Fc-Region auf die Nummerierung gemäß dem EU-Index, oder entsprechend in Kabat, bezieht.

2. Verfahren nach Anspruch 1, wobei die Aminosäuremodifikation 293Del die einzige Aminosäuremodifikation ist, die in die Fc-Region des Vorläuferpolypeptids eingeführt ist.

3. Verfahren nach Anspruch 1, wobei die Aminosäuremodifikation del293/del294 die einzige Aminosäuremodifikation ist, die in der Fc-Region des Vorläuferpolypeptids eingeführt ist.

4. Verfahren nach einem der Ansprüche 1 bis 3, wobei die Fc-Region des Vorläuferpolypeptids eine Variante einer Wildtyp-IgG-Fc-Region ist, die eine Aminosäuremodifikation ausgewählt aus 434Y, 378V, 259I/315D/434Y und 256N/378V/383N/434Y umfasst.

5. Verfahren nach einem der Ansprüche 1 bis 4, wobei die Variante ausgewählt ist aus der Gruppe bestehend aus isolierten Fcs, Fc-Fusionsproteinen, Fc-Konjugaten und Antikörpern.

6. Verfahren nach einem der Ansprüche 1 bis 5, wobei der Schritt des Einführens von besagter Aminosäuremodifikation in die Fc-Region des Vorläuferpolypeptids umfasst:
(i) Bereitstellen einer Nukleinsäure, die das Vorläuferpolypeptid kodiert,
(ii) Modifizieren der in Schritt (i) bereitgestellten Nukleinsäure, um eine Nukleinsäure zu erhalten, die für die genannte Variante kodiert, und
(iii) Exprimieren der in Schritt (ii) erhaltenen Nukleinsäure in einer Wirtszelle und Zurückerlangen der genannten Variante.

7. Variante eines Vorläuferpolypeptids, die eine Fc-Region der SEQ ID NO:1 umfasst und im Vergleich zu dem genannten Vorläuferpolypeptid eine reduzierte Bindung an das Protein C1q und an mindestens einen FcgR-Rezeptor ausgewählt aus FcγRII und FcγRIII aufweist, und wobei ein Aminosäuremodifikation bestehend aus 293del/294del in die Fc-Region des Vorläuferpolypeptids eingeführt ist, wobei sich die Nummerierung der Aminosäuren in der Fc-Region auf die Nummerierung gemäß dem EU-Index, oder entsprechend in Kabat, bezieht.

8. Variante nach Anspruch 7, wobei die Variante ein neutralisierender Antikörper ist, der auf ein Zielmolekül gerichtet ist, das ausgewählt ist aus der Gruppe bestehend aus Membranrezeptoren, menschliche lösliche Proteine, Toxine, virale, bakterielle und mykotische Proteine.

9. Isolierte Nukleinsäure, die für eine Variante gemäß einem der Ansprüche 7 bis 10 kodiert.

10. Vektor, umfassend die Nukleinsäure nach Anspruch 9.

11. Wirtszelle, die den Vektor nach Anspruch 10 enthält.

12. Variante eines Vorläuferpolypeptids zur Verwendung bei der Vorbeugung oder Behandlung eines pathologischen Zustands, wobei die Induktion von einer Antikörper-abhängigen, zellvermittelten Zytotoxizität (ADCC) und/oder einer Komplement-abhängigen Zytotoxizitäts-(CDC-)Antwort nicht erwünscht ist, wobei besagte Variante eine Fc-Region umfasst, im Vergleich zum genannten Vorläuferpolypeptid eine reduzierte Bindung an das Protein C1q und an mindestens einen Rezeptor FcgR aufweist, und innerhalb ihrer Fc-Region eine Aminosäuremodifikation umfasst, die gegenüber der Fc-Region von SEQ ID NO: 1 ihres Vorläuferpolypeptids ausgewählt ist aus 294Del, 293Del und 293Del/294Del, mit der Maßgabe, dass diese Variante des Vorläuferpolypeptids nicht aus den Varianten 294Del/T307P/N434Y oder 293Del/T307P/N434Y besteht, wobei sich die Nummerierung der Aminosäuren in der Fc-Region auf die Nummerierung gemäß dem EU-Index, oder entsprechend in Kabat, bezieht.

13. Pharmazeutische Zusammensetzung, enthaltend eine Variante, wie sie in den Ansprüchen 7 oder 8 definiert ist.

## Revendications

1. Procédé pour produire un variant d'un polypeptide parent comprenant une région Fc de SEQ ID NO: 1, lequel variant exhibe une liaison réduite à la protéine C1q et à au moins un récepteur FcgR par comparaison audit polypeptide parent, dans lequel une modification d'acide aminé sélectionnée dans le groupe consistant en :
(i) 294Del, où ladite modification d'acide aminé 294deI est la seule modification d'acide aminé introduite dans la région Fc du polypeptide parent,
(ii) 293Del, et
(iii) 293Del/294Del,
est introduite au sein de la région Fc du polypeptide parent, la numérotation des acides aminés dans la région Fc se référant à la numérotation selon l'index UE, ou équivalente dans Kabat.

2. Procédé selon la revendication 1, dans lequel la modification d'acide aminé 293Del est la seule modification d'acide aminé introduite dans la région Fc du polypeptide parent.

3. Procédé selon la revendication 1, dans lequel la modification d'acide aminé del293/del294 est la seule modification d'acide aminé introduite dans la région Fc du polypeptide parent.

4. Procédé selon l'une quelconque des revendications 1 à 3, dans lequel la région Fc du polypeptide parent est un variant d'une région Fc d'IgG de type sauvage comprenant une modification d'acide aminé sélectionnée parmi 434Y, 378V, 259I/315D/434Y et 256N/378V/383N/434Y.

5. Procédé selon l'une quelconque des revendications 1 à 4, dans lequel le variant est sélectionné dans le groupe consistant en des Fc isolés, des protéines de fusion avec Fc, des conjugués avec Fc et des anticorps.

6. Procédé selon l'une quelconque des revendications 1 à 5, dans lequel l'étape d'introduction de ladite modification d'acide aminé au sein de la région Fc du polypeptide parent comprend :
(i) la mise à disposition d'un acide nucléique codant pour le polypeptide parent,
(ii) la modification de l'acide nucléique mis à disposition à l'étape (i) de sorte à obtenir un acide nucléique codant pour ledit variant, et
(iii) l'expression de l'acide nucléique obtenu à l'étape (ii) dans une cellule hôte et la récupération dudit variant.

7. Variant d'un polypeptide parent comprenant une région Fc de SEQ ID NO: 1, et exhibant une liaison réduite à la protéine C1q et à au moins un récepteur FcgR choisi parmi FcγRII et FcγRIII par comparaison audit polypeptide parent, et dans lequel une modification d'acide aminé consistant en 293del/294del est introduite au sein de la région Fc du polypeptide parent, la numérotation des acides aminés dans la région Fc se référant à la numérotation selon l'index UE, ou équivalente dans Kabat.

8. Variant selon la revendication 7, où le variant est un anticorps neutralisant dirigé contre une molécule cible sélectionnée dans le groupe consistant en des récepteurs membranaires, des protéines solubles humaines, des toxines, des protéines virales, bactériennes et fongiques.

9. Acide nucléique isolé codant pour un variant tel que défini dans l'une quelconque des revendications 7 à 8.

10. Vecteur comprenant l'acide nucléique selon la revendication 9.

11. Cellule hôte contenant le vecteur selon la revendication 10.

12. Variant d'un polypeptide parent pour son utilisation dans la prévention ou le traitement d'une affection pathologique dans laquelle l'induction d'une réponse de cytotoxicité à médiation cellulaire dépendante des anticorps (ADCC) et/ou de cytotoxicité dépendante du complément (CDC) n'est pas souhaitable, ledit variant comprenant une région Fc, exhibant une liaison réduite à la protéine C1q et à au moins un récepteur FcgR par comparaison audit polypeptide parent et comprenant une modification d'acide aminé sélectionnée parmi 294Del, 293Del et 293Del/294Del au sein de sa région Fc, par comparaison à la région Fc de SEQ ID NO: 1 de son polypeptide parent, à condition que ledit variant du polypeptide parent ne consiste pas en les variants 294Del/T307P/N434Y ou 293Del/T307P/N434Y, la numérotation des acides aminés dans la région Fc se référant à la numérotation selon l'index UE, ou équivalente dans Kabat.

13. Composition pharmaceutique comprenant un variant tel que défini dans l'une quelconque des revendications 7 ou 8.


### SEQUENCE LISTING

<110> LFB Biotechnologies
   Millegen
<120> Fc variants with reduced effector functions
<130> X822EP
<160> 13
<170> PatentIn version 3.3
<210> 1
   <211> 222
   <212> PRT
   <213> Homo sapiens
<220>
   <221> MISC_FEATURE
   <222> (131)..(131)
   <223> For Glm1,17 allotype, x is D
<220>
   <221> MISC_FEATURE
   <222> (131)..(131)
   <223> For Glm3 allotype, x is E
<220>
   <221> MISC_FEATURE
   <222> (133)..(133)
   <223> For Glm1,17 allotype, x is L
<220>
   <221> MISC_FEATURE
   <222> (133)..(133)
   <223> For Glm3 allotype, x is M
<400> 1
   Cys Pro Pro Cys Pro Ala Pro Glu Leu Leu Gly Gly Pro Ser Val Phe
   1               5                   10                  15
   Leu Phe Pro Pro Lys Pro Lys Asp Thr Leu Met Ile Ser Arg Thr Pro
               20                  25                  30
   Glu Val Thr Cys Val Val Val Asp Val Ser His Glu Asp Pro Glu Val
           35                  40                  45
   Lys Phe Asn Trp Tyr Val Asp Gly Val Glu Val His Asn Ala Lys Thr
       50                  55                  60
   Lys Pro Arg Glu Glu Gln Tyr Asn Ser Thr Tyr Arg Val Val Ser Val
   65                  70                  75                  80
   Leu Thr Val Leu His Gln Asp Trp Leu Asn Gly Lys Glu Tyr Lys Cys
                   85                  90                  95
   Lys Val Ser Asn Lys Ala Leu Pro Ala Pro Ile Glu Lys Thr Ile Ser
   100 105 110
   Lys Ala Lys Gly Gln Pro Arg Glu Pro Gln Val Tyr Thr Leu Pro Pro
   115 120 125
   Ser Arg Xaa Glu Xaa Thr Lys Asn Gln Val Ser Leu Thr Cys Leu Val
   130 135 140
   Lys Gly Phe Tyr Pro Ser Asp Ile Ala Val Glu Trp Glu Ser Asn Gly
   145 150 155 160
   Gln Pro Glu Asn Asn Tyr Lys Thr Thr Pro Pro Val Leu Asp Ser Asp
   165 170 175
   Gly Ser Phe Phe Leu Tyr Ser Lys Leu Thr Val Asp Lys Ser Arg Trp
   180 185 190
   Gln Gln Gly Asn Val Phe Ser Cys Ser Val Met His Glu Ala Leu His
   195 200 205
   Asn His Tyr Thr Gln Lys Ser Leu Ser Leu Ser Pro Gly Lys
   210 215 220
<210> 2
   <211> 221
   <212> PRT
   <213> Homo sapiens
<400> 2
   Cys Pro Pro Cys Pro Ala Pro Pro Val Ala Gly Pro Ser Val Phe Leu
   1 5 10 15
   Phe Pro Pro Lys Pro Lys Asp Thr Leu Met Ile Ser Arg Thr Pro Glu
   20 25 30
   Val Thr Cys Val Val Val Asp Val Ser His Glu Asp Pro Glu Val Gln
   35 40 45
   Phe Asn Trp Tyr Val Asp Gly Val Glu Val His Asn Ala Lys Thr Lys
   50 55 60
   Pro Arg Glu Glu Gln Tyr Asn Ser Thr Tyr Arg Val Val Ser Val Leu
   65 70 75 80
   Thr Val Leu His Gln Asp Trp Leu Asn Gly Lys Glu Tyr Lys Cys Lys
   85 90 95
   Val Ser Asn Lys Ala Leu Pro Ala Pro Ile Glu Lys Thr Ile Ser Lys
   100 105 110
   Thr Lys Gly Gln Pro Arg Glu Pro Gln Val Tyr Thr Leu Pro Pro Ser
   115 120 125
   Arg Glu Glu Met Thr Lys Asn Gln Val Ser Leu Thr Cys Leu Val Lys
   130 135 140
   Gly Phe Tyr Pro Ser Asp Ile Ala Val Glu Trp Glu Ser Asn Gly Gln
   145 150 155 160
   Pro Glu Asn Asn Tyr Lys Thr Thr Pro Pro Met Leu Asp Ser Asp Gly
   165 170 175
   Ser Phe Phe Leu Tyr Ser Lys Leu Thr Val Asp Lys Ser Arg Trp Gln
   180 185 190
   Gln Gly Asn Val Phe Ser Cys Ser Val Met His Glu Ala Leu His Asn
   195 200 205
   His Tyr Thr Gln Lys Ser Leu Ser Leu Ser Pro Gly Lys
   210 215 220
<210> 3
   <211> 222
   <212> PRT
   <213> Homo sapiens
<400> 3
   Cys Pro Arg Cys Pro Ala Pro Glu Leu Leu Gly Gly Pro Ser Val Phe
   1 5 10 15
   Leu Phe Pro Pro Lys Pro Lys Asp Thr Leu Met Ile Ser Arg Thr Pro
   20 25 30
   Glu Val Thr Cys Val Val Val Asp Val Ser His Glu Asp Pro Glu Val
   35 40 45
   Gln Phe Lys Trp Tyr Val Asp Gly Val Glu Val His Asn Ala Lys Thr
   50 55 60
   Lys Pro Arg Glu Glu Gln Tyr Asn Ser Thr Phe Arg Val Val Ser Val
   65 70 75 80
   Leu Thr Val Leu His Gln Asp Trp Leu Asn Gly Lys Glu Tyr Lys Cys
   85 90 95
   Lys Val Ser Asn Lys Ala Leu Pro Ala Pro Ile Glu Lys Thr Ile Ser
   100 105 110
   Lys Thr Lys Gly Gln Pro Arg Glu Pro Gln Val Tyr Thr Leu Pro Pro
   115 120 125
   Ser Arg Glu Glu Met Thr Lys Asn Gln Val Ser Leu Thr Cys Leu Val
   130 135 140
   Lys Gly Phe Tyr Pro Ser Asp Ile Ala Val Glu Trp Glu Ser Ser Gly
   145 150 155 160
   Gln Pro Glu Asn Asn Tyr Asn Thr Thr Pro Pro Met Leu Asp Ser Asp
   165 170 175
   Gly Ser Phe Phe Leu Tyr Ser Lys Leu Thr Val Asp Lys Ser Arg Trp
   180 185 190
   Gln Gln Gly Asn Ile Phe Ser Cys Ser Val Met His Glu Ala Leu His
   195 200 205
   Asn Arg Phe Thr Gln Lys Ser Leu Ser Leu Ser Pro Gly Lys
   210 215 220
<210> 4
   <211> 221
   <212> PRT
   <213> Homo sapiens
<400> 4
   Cys Pro Ser Pro Ala Pro Glu Phe Leu Gly Gly Pro Ser Val Phe Leu
   1 5 10 15
   Phe Pro Pro Lys Pro Lys Asp Thr Leu Met Ile Ser Arg Thr Pro Glu
   20 25 30
   Val Thr Cys Val Val Val Asp Val Ser Gln Glu Asp Pro Glu Val Gln
   35 40 45
   Phe Asn Trp Tyr Val Asp Gly Met Glu Val His Asn Ala Lys Thr Lys
   50 55 60
   Pro Arg Glu Glu Gln Phe Asn Ser Thr Phe Arg Val Val Ser Val Leu
   65 70 75 80
   Thr Val Val His Gln Asp Trp Leu Asn Gly Lys Glu Tyr Lys Cys Lys
   85 90 95
   Val Ser Asn Lys Gly Leu Pro Ala Pro Ile Glu Lys Thr Ile Ser Lys
               100                 105                 110
   Ala Lys Gly Gln Pro Arg Glu Pro Gln Val Tyr Thr Leu Pro Pro Ser
           115                 120                 125
   Gln Glu Glu Met Thr Lys Asn Gln Val Ser Leu Thr Cys Leu Val Lys
       130                 135                 140
   Gly Phe Tyr Pro Ser Asp Ile Ala Val Glu Trp Glu Ser Asn Gly Gln
   145                 150                 155                 160
   Pro Glu Asn Asn Tyr Lys Thr Thr Pro Pro Val Leu Asp Ser Asp Gly
                   165                 170                 175
   Ser Phe Phe Leu Tyr Ser Arg Leu Thr Val Asp Lys Ser Arg Trp Gln
               180                 185                 190
   Glu Gly Asn Val Phe Ser Cys Ser Val Met His Glu Ala Leu His Asn
           195                 200                 205
   His Tyr Thr Gln Lys Ser Leu Ser Leu Ser Leu Gly Lys
       210                 215                 220
<210> 5
   <211> 232
   <212> PRT
   <213> Homo sapiens
<400> 5
   Glu Pro Lys Ser Cys Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
   100 105 110
   Leu Pro Ala Pro Ile Glu Lys Thr Ile Ser Lys Ala Lys Gly Gln Pro
   115 120 125
   Arg Glu Pro Gln Val Tyr Thr Leu Pro Pro Ser Arg Asp Glu Leu Thr
   130 135 140
   Lys Asn Gln Val Ser Leu Thr Cys Leu Val Lys Gly Phe Tyr Pro Ser
   145 150 155 160
   Asp Ile Ala Val Glu Trp Glu Ser Asn Gly Gln Pro Glu Asn Asn Tyr
   165 170 175
   Lys Thr Thr Pro Pro Val Leu Asp Ser Asp Gly Ser Phe Phe Leu Tyr
   180 185 190
   Ser Lys Leu Thr Val Asp Lys Ser Arg Trp Gln Gln Gly Asn Val Phe
   195 200 205
   Ser Cys Ser Val Met His Glu Ala Leu His Asn His Tyr Thr Gln Lys
   210 215 220
   Ser Leu Ser Leu Ser Pro Gly Lys
   225 230
<210> 6
   <211> 232
   <212> PRT
   <213> Homo sapiens
<400> 6
   Glu Pro Lys Ser Cys Asp Lys Thr His Thr Cys Pro Pro Cys Pro Ala
   1 5 10 15
   Pro Glu Leu Leu Gly Gly Pro Ser Val Phe Leu Phe Pro Pro Lys Pro
   20 25 30
   Lys Asp Thr Leu Met Ile Ser Arg Thr Pro Glu Val Thr Cys Val Val
   35 40 45
   Val Asp Val Ser His Glu Asp Pro Glu Val Lys Phe Asn Trp Tyr Val
   50 55 60
   Asp Gly Val Glu Val His Asn Ala Lys Thr Lys Pro Arg Glu Glu Gln
   65 70 75 80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Glu Glu Met Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Gly Leu His Asn His Tyr Thr Gln Lys
       210                 215                 220
   Ser Leu Ser Leu Ser Pro Gly Lys
   225                 230
<210> 7
   <211> 228
   <212> PRT
   <213> Homo sapiens
<400> 7
   Glu Arg Lys Cys Cys Val Glu Cys Pro Pro Cys Pro Ala Pro Pro Val
   1               5                   10                  15
   Ala Gly Pro Ser Val Phe Leu Phe Pro Pro Lys Pro Lys Asp Thr Leu
               20                  25                  30
   Met Ile Ser Arg Thr Pro Glu Val Thr Cys Val Val Val Asp Val Ser
           35                  40                  45
   His Glu Asp Pro Glu Val Gln Phe Asn Trp Tyr Val Asp Gly Val Glu
       50                  55                  60
   Val His Asn Ala Lys Thr Lys Pro Arg Glu Glu Gln Tyr Asn Ser Thr
   65 70 75 80
   Tyr Arg Val Val Ser Val Leu Thr Val Leu His Gln Asp Trp Leu Asn
   85 90 95
   Gly Lys Glu Tyr Lys Cys Lys Val Ser Asn Lys Ala Leu Pro Ala Pro
   100 105 110
   Ile Glu Lys Thr Ile Ser Lys Thr Lys Gly Gln Pro Arg Glu Pro Gln
   115 120 125
   Val Tyr Thr Leu Pro Pro Ser Arg Glu Glu Met Thr Lys Asn Gln Val
   130 135 140
   Ser Leu Thr Cys Leu Val Lys Gly Phe Tyr Pro Ser Asp Ile Ala Val
   145 150 155 160
   Glu Trp Glu Ser Asn Gly Gln Pro Glu Asn Asn Tyr Lys Thr Thr Pro
   165 170 175
   Pro Met Leu Asp Ser Asp Gly Ser Phe Phe Leu Tyr Ser Lys Leu Thr
   180 185 190
   Val Asp Lys Ser Arg Trp Gln Gln Gly Asn Val Phe Ser Cys Ser Val
   195 200 205
   Met His Glu Ala Leu His Asn His Tyr Thr Gln Lys Ser Leu Ser Leu
   210 215 220
   Ser Pro Gly Lys
   225
<210> 8
   <211> 279
   <212> PRT
   <213> Homo sapiens
<400> 8
   Glu Leu Lys Thr Pro Leu Gly Asp Thr Thr His Thr Cys Pro Arg Cys
   1 5 10 15
   Pro Glu Pro Lys Ser Cys Asp Thr Pro Pro Pro Cys Pro Arg Cys Pro
   20 25 30
   Glu Pro Lys Ser Cys Asp Thr Pro Pro Pro Cys Pro Arg Cys Pro Glu
   35 40 45
   Pro Lys Ser Cys Asp Thr Pro Pro Pro Cys Pro Arg Cys Pro Ala Pro
       50                  55                  60
   Glu Leu Leu Gly Gly Pro Ser Val Phe Leu Phe Pro Pro Lys Pro Lys
   65                  70                  75                  80
   Asp Thr Leu Met Ile Ser Arg Thr Pro Glu Val Thr Cys Val Val Val
                   85                  90                  95
   Asp Val Ser His Glu Asp Pro Glu Val Gln Phe Lys Trp Tyr Val Asp
               100                 105                 110
   Gly Val Glu Val His Asn Ala Lys Thr Lys Pro Arg Glu Glu Gln Tyr
           115                 120                 125
   Asn Ser Thr Phe Arg Val Val Ser Val Leu Thr Val Leu His Gln Asp
       130                 135                 140
   Trp Leu Asn Gly Lys Glu Tyr Lys Cys Lys Val Ser Asn Lys Ala Leu
   145                 150                 155                 160
   Pro Ala Pro Ile Glu Lys Thr Ile Ser Lys Thr Lys Gly Gln Pro Arg
                   165                 170                 175
   Glu Pro Gln Val Tyr Thr Leu Pro Pro Ser Arg Glu Glu Met Thr Lys
               180                 185                 190
   Asn Gln Val Ser Leu Thr Cys Leu Val Lys Gly Phe Tyr Pro Ser Asp
           195                 200                 205
   Ile Ala Val Glu Trp Glu Ser Ser Gly Gln Pro Glu Asn Asn Tyr Asn
       210                 215                 220
   Thr Thr Pro Pro Met Leu Asp Ser Asp Gly Ser Phe Phe Leu Tyr Ser
   225                 230                 235                 240
   Lys Leu Thr Val Asp Lys Ser Arg Trp Gln Gln Gly Asn Ile Phe Ser
                   245                 250                 255
   Cys Ser Val Met His Glu Ala Leu His Asn Arg Phe Thr Gln Lys Ser
               260                 265                 270
   Leu Ser Leu Ser Pro Gly Lys
           275
<210> 9
   <211> 228
   <212> PRT
   <213> Homo sapiens
<400> 9
   Glu Ser Lys Tyr Gly Pro Pro Cys Pro Ser Pro Ala Pro Glu Phe Leu
   1               5                   10                  15
   Gly Gly Pro Ser Val Phe Leu Phe Pro Pro Lys Pro Lys Asp Thr Leu
               20                  25                  30
   Met Ile Ser Arg Thr Pro Glu Val Thr Cys Val Val Val Asp Val Ser
           35                  40                  45
   Gln Glu Asp Pro Glu Val Gln Phe Asn Trp Tyr Val Asp Gly Met Glu
       50                  55                  60
   Val His Asn Ala Lys Thr Lys Pro Arg Glu Glu Gln Phe Asn Ser Thr
   65                  70                  75                  80
   Phe Arg Val Val Ser Val Leu Thr Val Val His Gln Asp Trp Leu Asn
                   85                  90                  95
   Gly Lys Glu Tyr Lys Cys Lys Val Ser Asn Lys Gly Leu Pro Ala Pro
               100                 105                 110
   Ile Glu Lys Thr Ile Ser Lys Ala Lys Gly Gln Pro Arg Glu Pro Gln
           115                 120                 125
   Val Tyr Thr Leu Pro Pro Ser Gln Glu Glu Met Thr Lys Asn Gln Val
       130                 135                 140
   Ser Leu Thr Cys Leu Val Lys Gly Phe Tyr Pro Ser Asp Ile Ala Val
   145                 150                 155                 160
   Glu Trp Glu Ser Asn Gly Gln Pro Glu Asn Asn Tyr Lys Thr Thr Pro
                   165                 170                 175
   Pro Val Leu Asp Ser Asp Gly Ser Phe Phe Leu Tyr Ser Arg Leu Thr
               180                 185                 190
   Val Asp Lys Ser Arg Trp Gln Glu Gly Asn Val Phe Ser Cys Ser Val
           195                 200                 205
   Met His Glu Ala Leu His Asn His Tyr Thr Gln Lys Ser Leu Ser Leu
       210                 215                 220
   Ser Leu Gly Lys
                           225
<210> 10
   <211> 35
   <212> DNA
   <213> Artificial
<220>
   <223> 5'-Primer
<400> 10
   agtactgact ctacctagga tcctgcccac cgtgc 35
<210> 11
   <211> 24
   <212> DNA
   <213> Artificial
<220>
   <223> 3'-Primer
<400> 11
   gctgttgtac tgctcccgcg gctt 24
<210> 12
   <211> 24
   <212> DNA
   <213> Artificial
<220>
   <223> 5'-Primer
<400> 12
   aagccgcggg agcagtacaa cagc 24
<210> 13
   <211> 35
   <212> DNA
   <213> Artificial
<220>
   <223> 3'-Primer
<400> 13
   actgctcgat gtccgtacta tgcggccgcg aattc 35
1